# EUROPÄISCHE PATENTSCHRIFT

(11) **EP 2 872 524 B1**
(45) Veröffentlichungstag und Bekanntmachung des Hinweises auf die Patenterteilung: **24.08.2016**
(21) Anmeldenummer: 13734744.9
(22) Anmeldetag: 05.07.2013
(51) Int. Cl.: C07J 43/00, A61K 31/58, A61P 5/28, A61P 5/32

(54) **3-SUBSTITUIERTE ESTRA-1,3,5 (10),16-TETRAEN-DERIVATE, VERFAHREN ZU IHRER HERSTELLUNG, PHARMAZEUTISCHE PRÄPARATE DIE DIESE ENTHALTEN, SOWIE DEREN VERWENDUNG ZUR HERSTELLUNG VON ARZNEIMITTELN**
3-SUBSTITUTED ESTRA-1,3,5(10),16-TETRAENE DERIVATIVES, METHODS FOR THE PRODUCTION THEREOF, PHARMACEUTICAL PREPARATIONS CONTAINING SAME, AND USE THEREOF FOR THE PRODUCTION OF MEDICAMENTS
DÉRIVÉS D'OESTRA-1,3,5 (10), 16-TÉTRAÈNE SUBSTITUÉS EN POSITION 3, LEURS PROCÉDÉS DE PRÉPARATION, PRÉPARATIONS PHARMACEUTIQUES LES CONTENANT, ET LEUR UTILISATION POUR LA FABRICATION DE MÉDICAMENTS

(30) Priorität: 10.07.2012 DE 102012211970
(43) Veröffentlichungstag der Anmeldung: 20.05.2015
(73) Patentinhaber: Bayer Pharma Aktiengesellschaft, 13353 Berlin (DE)
(72) Erfinder: BOTHE, Ulrich, 13187 Berlin (DE); BUSEMANN, Matthias, 13467 Berlin (DE); FISCHER, Oliver Martin, 13127 Berlin (DE); BARAK, Naomi, 12049 Berlin (DE); ROTGERI, Andrea, 13503 Berlin (DE); MARQUARDT, Tobias, 42115 Wuppertal (DE); STEGMANN, Christian, 10437 Berlin (DE)
(74) Vertreter: BIP Patents
(86) Internationale Anmeldenummer: PCT/EP2013/064257
(87) Internationale Veröffentlichungsnummer: WO 2014/009274

(56) Entgegenhaltungen:
- WO-A1-2013/045407
- WO-A2-00/07576
- WO-A2-99/46279
- MOSTAGHEL ELAHE A ET AL: "Resistance to CYP17A1 inhibition with abiraterone in castration-resistant prostate cancer: induction of steroidogenesis and androgen receptor splice variants.", CLINICAL CANCER RESEARCH : AN OFFICIAL JOURNAL OF THE AMERICAN ASSOCIATION FOR CANCER RESEARCH 15 SEP 2011, Bd. 17, Nr. 18, 15. September 2011 (2011-09-15), Seiten 5913-5925, XP002712865, ISSN: 1078-0432
- LI RUI ET AL: "Abiraterone inhibits 3[beta]-hydroxysteroid dehydrogenase: a rationale for increasing drug exposure in castration-resistant prostate cancer.", CLINICAL CANCER RESEARCH : AN OFFICIAL JOURNAL OF THE AMERICAN ASSOCIATION FOR CANCER RESEARCH 1 JUL 2012, Bd. 18, Nr. 13, 1. Juli 2012 (2012-07-01), Seiten 3571-3579, XP002712888, ISSN: 1078-0432

## Beschreibung

Die Erfindung betrifft AKR1C3 Inhibitoren und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Blutungsbeschwerden und Endometriose.

AKR1C3 ist ein multifunktionales Enzym und katalysiert unter anderem die Reduktion von 4-Androsten-3,17-dion (einem schwachen Androgen) zu Testosteron (einem potenten Androgen) und von Estron (einem schwachen Estrogen) zu 17β-Estradiol (einem starken Estrogen). Zusätzlich wird die Reduktion von Prostaglandin (PG) H2 zu PGF2α und PGD2 zu 9α,11β-PGF2 inhibiert (T. M. Penning et. al., 2006, 'Aldo-keto reductase (AKR) 1C3: Role in prostate disease and the development of specific inhibitors', Molecular and Cellular Endocrinology 248(1-2), 182 -191).

Die lokale Bildung von Estradiol (E2) spielt eine zentrale Rolle für die Initiierung und das Fortschreiten von Brustkrebserkrankungen und Endometriose. Die Reduktion der Gewebespiegel von Estrogenen und insbesondere von Estradiol wird erreicht durch die therapeutische Gabe von Aromataseinhibitoren (um die Bildung von Estrogenen aus Androgenen zu inhibieren) und von Sulfataseinhibitoren (um die Bildung von Estron aus Estronsulfat zu blockieren). Beide Therapieansätze haben aber den Nachteil, dass systemische Estrogenspiegel radikal reduziert werden (A. Oster et. al., J. Med. Chem. 2010, 53, 8176-8186). Kürzlich wurde experimentell nachgewiesen, dass endometriotische Läsionen in der Lage sind lokal Estradiol zu synthetisieren (B. Delvoux et al., J Clin Endocrinol Metab. 2009, 94, 876-883). Für den Subtyp der ovariellen Endometriose wurde eine Überexpression der AKR1C3 mRNA beschrieben (T. Smuc et al., Mol Cell Endocrinol. 2009 Mar 25;301(1-2): 59-64).

An der Identifizierung von neuen Inhibitoren des Enzyms Aldo-Keto-Reduktase 1C3 (AKR1C3; Synonyme: Typ 5 17β-Hydroxysteroid Dehydrogenase oder Prostaglandin F Synthase) besteht großer Bedarf, da Inhibitoren ein Potential für die Behandlung von hormonabhängigen Erkrankungen wie zum Beispiel Endometriose aber auch für die Behandlung von hormonunabhängigen Erkrankungen haben (M.C. Byrns, Y. Jin, T.M. Penning, Journal of Steroid Biochemistry and Molecular Biology (2010); A. L. Lovering et. al., *Cancer Res* **64**(5), 1802-1810). Neben der Endometriose zählen dazu auch Prostatakrebs (K. M. Fung et al., Endocr Relat Cancer 13(1), 169-180), Prostatahyperplasie (R. O. Roberts et al., Prostate 66(4), 392-404), Endometriumskarzinom (T. L. Rizner et al., Mol Cell Endocrinol 2006 248(1-2), 126-135), polyzystisches ovarielles Syndrom (K. Qin et al., J Endocrinol. Metab 2006, 91(1), 270-276), Lungenkarzinom (Q. Lan et al., Corcinogenesis 2004, 25(11), 2177-2181), non-Hodgkin-Lymphom (Q. Lan et al., Hum Genet 2007, 121(2), 161-168), Haarausfall (L. Colombe et al., Exp Dermatol 2007, 16(9), 762-769), Adipositas (P. A. Svensson et al., Cell Mol Biol Lett 2008, 13(4), 599-613), Blasenkarzinom (J. D. Figueroa, Carcinogenesis 2008, 29(10), 1955-1962), chronische myeloische Leukämie (J. Birthwistle, Mutat Res 2009, 662(1-2), 67-74), Nierenzellkarzinom (J. T. Azzarello, Int J Clin Exp Patho/ 2009, 3(2), 147-155), Brustkrebs (M. C. Byrns, J Steroid Biochem Mol Biol 2010, 118(3), 177-187), die frühzeitige Geschlechtsreife (C. He, Hum Genet 2010, 128(5), 515-527) und die chronisch-obstruktive Lungenerkrankung (S. Pierrou, Am J Respir Crit Care 2007, 175(6), 577-586).

Einige Inhibitoren von AKR1C3 sind bekannt (Übersichtsartikel: Joanna M Day, Helena J Tutill, Atul Purohit und Michael J Reed, Endocrine-Related Cancer (2008) 15, 665-692, siehe auch Patente US20100190826 sowie WO2007/100066). Als steroidale Substanz wurde zum Beispiel EM-1404 basierend auf dem Estratrien-Gerüst mit einer Spirolactoneinheit an Position 17 beschrieben (F. Labrie et al. US Patent 6,541,463, 2003).

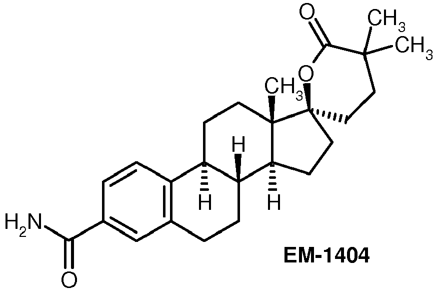

Weitere steroidale AKR1C3 Inhibitoren mit Lactoneinheit findet man in P. Bydal, Van Luu-The, F. Labrie, D. Poirier, European Journal of Medicinal Chemistry 2009, 44, 632-644. Fluorierte Estratrienderivate wurden beschrieben in D. Deluca, G. Moller, A. Rosinus, W. Elger, A. Hillisch, J. Adamski, Mol. Cell. Endocrinol. 2006, 248, 218 - 224.

Bei den erfindungsgemäßen Verbindungen handelt es sich um Substanzen basierend auf einem Estra-1,3,5(10),16-tetraen-Gerüst substituiert mit einem aromatischen Heterocyclus an Position 17. In S. E. Barrie et al. US 5604213 wird dagegen 17-(3-Pyridyl)estra-1,3,5(10),16-tetraen-3-ol, eine verwandte Substanz die am Kohlenstoffatom 3 durch eine freie Hydroxygruppe substituiert ist, nur als 17α-Hydroxylase/C17-20 Lyase (Cyp17A1) Inhibitor beschrieben.

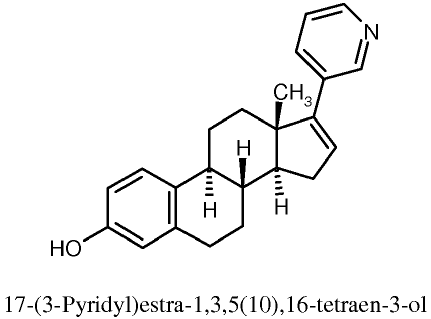

Es werden darüber hinaus in US 5604213 keine 17-(3-Pyridyl)estra-1,3,5(10),16-tetraen-Derivate beschrieben, die an Position 3 mit einer Alkoxy- oder einer Alkylgruppe substituiert sind. Die Substituenten an 3-Position der in der vorliegenden Erfindung beanspruchten, erfindungsgemäßen Verbindungen enthalten zusätzlich eine oder mehrere funktionelle Gruppen wie zum Beispiel Carboxylgruppen oder Hydroxylgruppen, wodurch sich ein weiterer struktureller Unterschied zu den in US 5604213 beschriebenen Substanzen ergibt. Überaschenderweise wurde nun gefunden, dass die hier beanspruchten, erfindungsgemäßen Verbindungen potente Inhibitoren von AKR1C3 sind.

Estra-1,3,5(10),16-tetraen-Derivate substituiert mit einer Aminocarbonyl (-CONH₂) Gruppe an 3-Position werden als antiproliferativ und antiangiogenetisch wirksam ohne Bezug auf ein konkretes molekulares Target beschrieben in US2005/0203075. Diese Derivate sind jedoch nicht mit einem Heterocyclus an Position 17 des Estra-1,3,5(10),16-tetraen-Gerüstes substituiert.

Eine Übersicht über 17-Pyridyl- und 17-Pyrimidylandrostan-Derivate, die als Cyp17A1 Inhibitoren beschrieben werden, findet man in V. M. Moreira et al. Current Medicinal Chemistry, 2008 Vol. 15, No. 9.

Erfindungsgemäße Verbindungen sind die Verbindungen der Formel (I) und deren Salze, Solvate und Solvate der Salze, sowie die von Formel (I) umfassten, nachfolgend als Ausführungsbeispiele genannten Verbindungen und deren Salze, Solvate und Solvate der Salze.

Gegenstand der vorliegenden Erfindung sind Verbindungen der Formel (I) 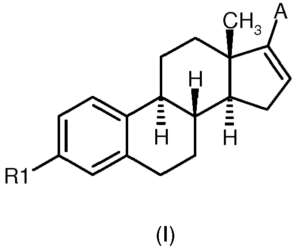 worin
- A: Pyridin-3-yl, Pyrimidin-5-yl, Pyrimidin-4-yl, Pyrazin-2-yl, Pyridazin-4-yl, Pyridazin-3-yl, gegebenenfalls einfach oder zweifach substituiert mit Fluor, Chlor, Nitril, Hydroxyl, Carboxyl, Trifluormethyl, Pentafluorethyl, Methoxy, Ethoxy, Trifluormethoxy, -OCH₂CF₃, -SO₂CH₃, -SO₂CH₂CH₃, -(C=O)CH₃, C₁-C₄-Alkyl, -CH₂OH, -C(CH₃)₂OH, -CONH₂, -(C=O)NHCH₃, -(C=O)NHCH₂CH₃, -(C=O)N(CH₃)₂, -SO₂NH₂, -SO₂NHCH₃, -SO₂N(CH₃)₂,
- R1: -O-CR^{a}R^{b}-Y, mit
R^{a} und R^{b} unabhängig voneinander Wasserstoff, Methyl, Ethyl, Propyl, Isopropyl, Phenyl, 4-Fluorphenyl, 3-Fluorphenyl, 2-Fluorphenyl, CH₃-O-CH₂-, -CH₂CF₃ oder
   R^{a} und R^{b} gemeinsam -(CH₂)ₙ- mit n = 2, 3, 4 oder 5 oder
   R^{a} und R^{b} gemeinsam -CH₂-O-CH₂-, -CH₂-NH-CH₂-, -CH₂-N(CH₃)-CH₂-, -CH₂CH₂-O-CH₂CH₂-, -CH₂CH₂-NH-CH₂CH₂-, -CH₂CH₂-N(CH₃)-CH₂CH₂--O-CR^{c}R^{d}-CR^{e}R^{f}-Y, mit

R^{c}, R^{d}, R^{e} R^{f} Wasserstoff oder
   R^{e}, R^{f} Wasserstoff und R^{c}, R^{d} unabhängig voneinander Methyl, Ethyl oder gemeinsam -(CH₂)ₙ- mit n = 2, 3, 4, 5 oder gemeinsam -CH₂-O-CH₂- oder -CH₂CH₂-O-CH₂CH₂- oder
   R^{c}, R^{d} Wasserstoff und R^{e}, R^{f} unabhängig voneinander Methyl, Ethyl, CF₃CH₂- oder gemeinsam -(CH₂)n- mit n = 2, 3, 4, 5, -CH₂CH₂-O-CH₂CH₂-, -CH₂CH₂-NH-CH₂CH₂-, -CH₂CH₂-N(CH₃)-CH₂CH₂-, -CH₂-O-CH₂-, -CH₂-NH-CH₂-, -CH₂-N(CH₃)-CH₂- oder
   R^{d}, R^{e}, R^{f} Wasserstoff und R^{c} Methyl, Ethyl, Trifluormethyl oder
   R^{c}, R^{d}, R^{f} Wasserstoff und R^{e} Methyl, Ethyl, Trifluormethyl, Hydroxyl, Methoxy, Trifluormethoxy oder
   R^{d}, R^{f} Wasserstoff und R^{c}, R^{e} unabhängig voneinander Methyl, Ethyl, Trifluormethyl,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂-CH(OH)-CH₂-Y
-OCH₂CH₂CH₂CH-Y,
-CH₂-Y,
-CR^{g}R^{h}-CRⁱR^{j}-Y, mit
R^{g,} R^{h,} Rⁱ, R^{j} Wasserstoff oder
   R^{g}, R^{h}, Rⁱ Wasserstoff und R^{j} Methyl, Ethyl, Trifluormethyl oder
   Rⁱ, R^{j} Wasserstoff und R^{g}, R^{h} Methyl oder gemeinsam -CH₂-, -CH₂CH₂-, -CH₂CH₂CH₂- oder
R^{g} Methyl und R^{h}, Rⁱ, R^{j} Wasserstoff,
-CH₂CH₂CH₂-Y,
-CH₂CH₂C(CH₃)₂-Y oder
-CH₂CH₂CH₂CH₂-Y und
- Y: -CO₂H, -OH, -(C=O)NH₂, -(C=O)NHC₁₋₄Alkyl, -S(=O)CH₃
bedeutet und deren Salze, Solvate und Solvate der Salze.

Bevorzugt sind Verbindungen der Formel (I) worin
- A: Pyridin-3-yl, Pyrimidin-5-yl, Pyrimidin-4-yl, Pyrazin-2-yl, Pyridazin-4-yl, Pyridazin-3-yl, gegebenenfalls einfach substituiert mit Fluor, Chlor, Nitril, Hydroxyl, Carboxyl, Trifluormethyl, Methoxy, Ethoxy, Trifluormethoxy, -SO₂CH₃, -(C=O)CH₃, C₁-C₄-Alkyl,
- R1: -O-CR^{a}R^{b}-Y, mit
R^{a} und R^{b} unabhängig voneinander Wasserstoff, Methyl, Ethyl, Propyl, Isopropyl, Phenyl, CH₃-O-CH₂-, CF₃CH₂-,
-O-CR^{c}R^{d}-CR^{e}R^{f}-Y, mit
R^{c}, R^{d}, R^{e} R^{f} Wasserstoff oder
   R^{c}, R^{d} Wasserstoff und Re, Rf unabhängig voneinander Methyl, Ethyl, CF₃CH₂- oder
   R^{d}, R^{e}, R^{f} Wasserstoff und R^{c} Methyl, Ethyl oder
   R^{c}, R^{d}, R^{f} Wasserstoff und R^{e} Methyl, Ethyl,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂-CH(OH)-CH2-Y oder
-CR^{g}R^{h}-CRⁱR^{j}-Y, mit
R^{g,} R^{h,} Rⁱ, R^{j} Wasserstoff oder
   R^{g}, R^{h}, Rⁱ Wasserstoff und R^{j} Methyl, Ethyl oder
   Rⁱ, R^{j} Wasserstoff und R^{g}, R^{h} Methyl oder
   R^{g} Methyl und R^{h}, Rⁱ, R^{j} Wasserstoff und
- Y: -CO₂H, -OH, -(C=O)NH₂, -(C=O)NHC₁₋₄Alkyl
bedeutet und deren Salze, Solvate und Solvate der Salze.

Besonders bevorzugt sind Verbindungen der Formel (I) worin
- A: Pyridin-3-yl, Pyrimidin-5-yl, Pyridazin-4-yl, gegebenenfalls einfach substituiert mit Fluor, Carboxyl, Trifluormethyl, Methyl
- R1: -O-CR^{a}R^{b}-Y, mit
R^{a} und R^{b} Wasserstoff oder R^{a} Wasserstoff und R^{b} Methyl, Ethyl, Phenyl oder CH₃-O-CH₂-
-O-CR^{c}R^{d}-CR^{e}R^{f}-Y, mit
R^{c}, R^{d} Wasserstoff und R^{e}, R^{f} Methyl
-O-CH₂CH₂CH₂-Y,
-O-CH₂C(CH₃)₂CH₂-Y,
-O-CH₂-CH(OH)-CH2-Y oder
-CH₂-CH₂-Y und
- Y: -CO₂H, -OH, -(C=O)NH₂, -(C=O)NHC₁₋₄Alkyl
bedeutet und deren Salze, Solvate und Solvate der Salze.

Ganz besonders bevorzugt sind Verbindungen der Formel (I) worin
- A: 5-Fluorpyridin-3-yl, 5-(Trifluormethyl)pyridin-3-yl, 5-Carboxypyridin-3-yl, 6-Methylpyridin-3-yl, Pyrimidin-5-yl, 6-Methylpyridazin-4-yl und
- R1: -O-CR^{a}R^{b}-CO₂H,
-O-CR^{a}R^{b}-(C=O)NH₂,
-O-CR^{a}R^{b}-(C=O)NHCH₂CH₃ mit
R^{a} und R^{b} Wasserstoff oder
   R^{a} Wasserstoff und R^{b} Methyl, Ethyl, Phenyl, CH₃OCH₂-,
-O-CR^{c}R^{d}-CR^{e}R^{f}-CO₂H mit
R^{c}, R^{d} Wasserstoff und R^{e}, R^{f} Methyl,
-O-CH₂CH₂CH₂-OH,
-O-CH₂CH₂CH₂-CO₂H,
-O-CH₂C(CH₃)₂CH₂-OH oder
-O-CH₂-CH(OH)-CH₂-OH
bedeutet und deren Salze, Solvate und Solvate der Salze.

Weiterhin sind Gegenstand der Erfindung die Verbindungen 3-[17-(5-Fluorpyridin-3-yl)estra-1,3,5(10),16-tetraen-3-yl]propansäure ({17-[5-(Trifluormethyl)pyridin-3-yl]estra-1,3,5(10),16-tetraen-3-yl}oxy)essigsäure {[17-(5-Fluorpyridin-3-yl)estra-1,3,5(10),16-tetraen-3-yl]oxy}essigsäure {[17-(6-Methylpyridin-3-yl)estra-1,3,5(10),16-tetraen-3-yl]oxy}essigsäure {[17-(Pyrimidin-5-yl)estra-1,3,5(10),16-tetraen-3-yl]oxy}essigsäure 5-[3-(Carboxymethoxy)estra-1,3,5(10),16-tetraen-17-yl]nicotinsäure {[17-(6-Methylpyridazin-4-yl)estra-1,3,5(10),16-tetraen-3-yl]oxy}essigsäure 3-{[17-(5-Fluorpyridin-3-yl)estra-1,3,5(10),16-tetraen-3-yl]oxy}-2,2-dimethylpropansäure 4-{[17-(5-Fluorpyridin-3-yl)estra-1,3,5(10),16-tetraen-3-yl]oxy}butansäure 2-{[17-(5-Fluorpyridin-3-yl)estra-1,3,5(10),16-tetraen-3-yl]oxy}propansäure 2-{[17-(5-Fluorpyridin-3-yl)estra-1,3,5(10),16-tetraen-3-yl]oxy}butansäure 2-{[17-(5-Fluorpyridin-3-yl)estra-1,3,5(10),16-tetraen-3-yl]oxy}-3-methoxypropansäure 2-{[17-(3-Pyridyl)estra-1,3,5(10),16-tetraen-3-yl]oxy}propansäure 2-{[17-(5-Fluorpyridin-3-yl)estra-1,3,5(10),16-tetraen-3-yl]oxy}propanamid 3-{[17-(5-Fluorpyridin-3-yl)estra-1,3,5(10),16-tetraen-3-yl]oxy}propan-1-ol 3-{[17-(5-Fluorpyridin-3-yl)estra-1,3,5(10),16-tetraen-3-yl]oxy}-2,2-dimethylpropan-1-ol 3-{[17-(5-Fluorpyridin-3-yl)estra-1,3,5(10),16-tetraen-3-yl]oxy}propan-1,2-diol {[17-(5-Fluorpyridin-3-yl)estra-1,3,5(10),16-tetraen-3-yl]oxy}(phenyl)essigsäure N-Ethyl-2-({17-[5-(trifluormethyl)pyridin-3-yl]estra-1,3,5(10),16-tetraen-3-yl}oxy)acetamid und deren Salze, Solvate und Solvate der Salze.

Es wurde gefunden, dass die erfindungsgegenständlichen 3-substituierten Estra-1,3,5(10),16-tetraen-Derivate als AKR1C3 Inhibitoren wirken. Die beanspruchten Verbindungen zeigen eine starke Inhibition von AKR1C3 *in vitro* (IC₅₀-Werte < 200 nM) und überwiegend sogar IC₅₀-Werte < 50 nM.

Die erfindungsgemäßen Verbindungen (vergl. bspw. Beispiel 10, 11, 12, 13, 14, 17 und 18) können in Abhängigkeit von ihrer Struktur in bestimmten stereoisomeren Formen (Diastereomere) existieren. Die Erfindung umfasst deshalb die Diastereomeren und ihre jeweiligen Mischungen. Aus solchen Mischungen von Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Essigsäure, Ameisensäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
C₁-C₄-Alkyl steht für einen linearen oder verzweigten Alkylrest mit 1 bis 4 Kohlenstoffatomen, beispielhaft und vorzugsweise für Methyl, Ethyl, Propyl, Butyl, Isopropyl, tert-Butyl, Isobutyl.

Weiterer Gegenstand der Erfindung sind Verfahren zur Herstellung der erfindungsgemäßen Verbindungen der Formel (I). Die Herstellung der erfindungsgemäßen Verbindungen (I) kann durch die nachfolgenden Syntheseschemata verdeutlicht werden:
Eine Teilmenge der erfindungsgemäßen Verbindungen lässt sich wie exemplarisch an der Synthese von Beispiel 1 beschrieben, ausgehend von Estron, herstellen (Syntheseschema 1): Die Umsetzung von Estron zu Intermediat 1 ist literaturbekannt (Tetrahedron Letters, 2003, 44, 3071 - 3074 oder Journal of Medicinal Chemistry, 1986, 29, 692 - 698) oder kann durch die Umsetzung von Estron mit 1,1,2,2,3,3,4,4,4-Nonafluorbutan-1-sulfonylfluorid in THF in Gegenwart von Kaliumcarbonat erfolgen.

Intermediat 2 wird durch Umsetzung mit Acrylsäureethylester mittels der Heck-Reaktion (J. Org. Chem., 1972, 37(14), 2320-2322) hergestellt. Bevorzugt erfolgt die Umsetzung mit Acrylsäureethylester, Triethylamin, Tri-2-tolylphosphin und Palladium(II)acetat in Acetontril.

Intermediat 3 wird durch Hydrierung in Gegenwart von Palladium auf Kohlenstoff synthetisiert.

Die Umsetzung zu Intermediat 4 erfolgt mit Trifluormethansulfonsäureanhydrid oder *N,N-*Bis(trifluormethansulfonyl)anilin in Gegenwart einer Base wie Pyridin, 2,6-Dimethylpyridin oder 2,6-Di-*tert*-butylpyridin oder in Gegenwart eines tertiären Amins wie Triethylamin oder Diisopropylethylamin oder durch Verwendung von Alkalimetallhexamethylsilazanen oder Lithium-diisopropylamid (LDA) (J. Med. Chem., 1995, 38, 2463 - 2471, J. Org. Chem., 1985, 50, 1990 - 1992, J. Am. Chem. Soc., 2009, 131, 9014 - 9019, Archiv der Pharmazie (Weinheim, Germany), 2001, 334, 12, 373 - 374). Bevorzugt ist die Umsetzung mit Trifluormethansulfonsäureanhydrid in Gegenwart von 2,6-Di-*tert*-butylpyridin in Dichlormethan.

Die Herstellung des Intermediates 5 erfolgt mit Hilfe der dem Fachmann bekannten *Suzuki-*Reaktion. Hierfür wird das Intermediat 4 mit 5-Fluorpyridin-3-boronsäure oder mit einem entsprechenden Boronsäureester wie z.B. einem Boronsäurepinakolester, einem *MIDA* Boronat (D. M. Knapp et al. J. Am. Chem. Soc. 2009, 131, 6961) oder mit einem Trifluorboratsalz (G. A. Molander et al., J. Org. Chem. 2009, 74, 973) umgesetzt. Als Katalysatoren kommen eine Vielzahl an palladiumhaltigen Katalysatoren in Betracht, wie zum Beispiel Tetrakis(triphenylphosphin)palladium(0), Bis(triphenylphosphin)-palladium(II)dichlorid oder [1,3-Bis(2,6-diisopropylphenyl)imidazol-2-yliden](3-chlorpyridyl)palladium(II) dichlorid (CAS 905459-27-0). Alternativ kann eine Palladiumhaltige Quelle wie zum Beispiel Palladium(II)-acetat, Palladium(II)-chlorid oder Pd(dba)₂ in Kombination mit einem phosphorhaltigen Liganden wie zum Beispiel Triphenylphosphin, SPhos (D. M. Knapp et. al., J. Am. Chem. Soc. 2009, 131, 6961) oder RuPhos (G. A. Molander, J. Org. Chem. 2009, 74, 973) eingesetzt werden. Bevorzugt ist die Umsetzung mit 5-Fluorpyridin-3-boronsäure in Gegenwart von Bis(triphenylphosphin)palladium(II)chlorid.

Die Herstellung von Beispiel 1 ausgehend von Intermediat 5 erfolgt durch die dem Fachmann bekannte Verseifungsreaktion von Carbonsäureestern. Bevorzugt ist die Umsetzung mit Natronlauge in THF und Ethanol.

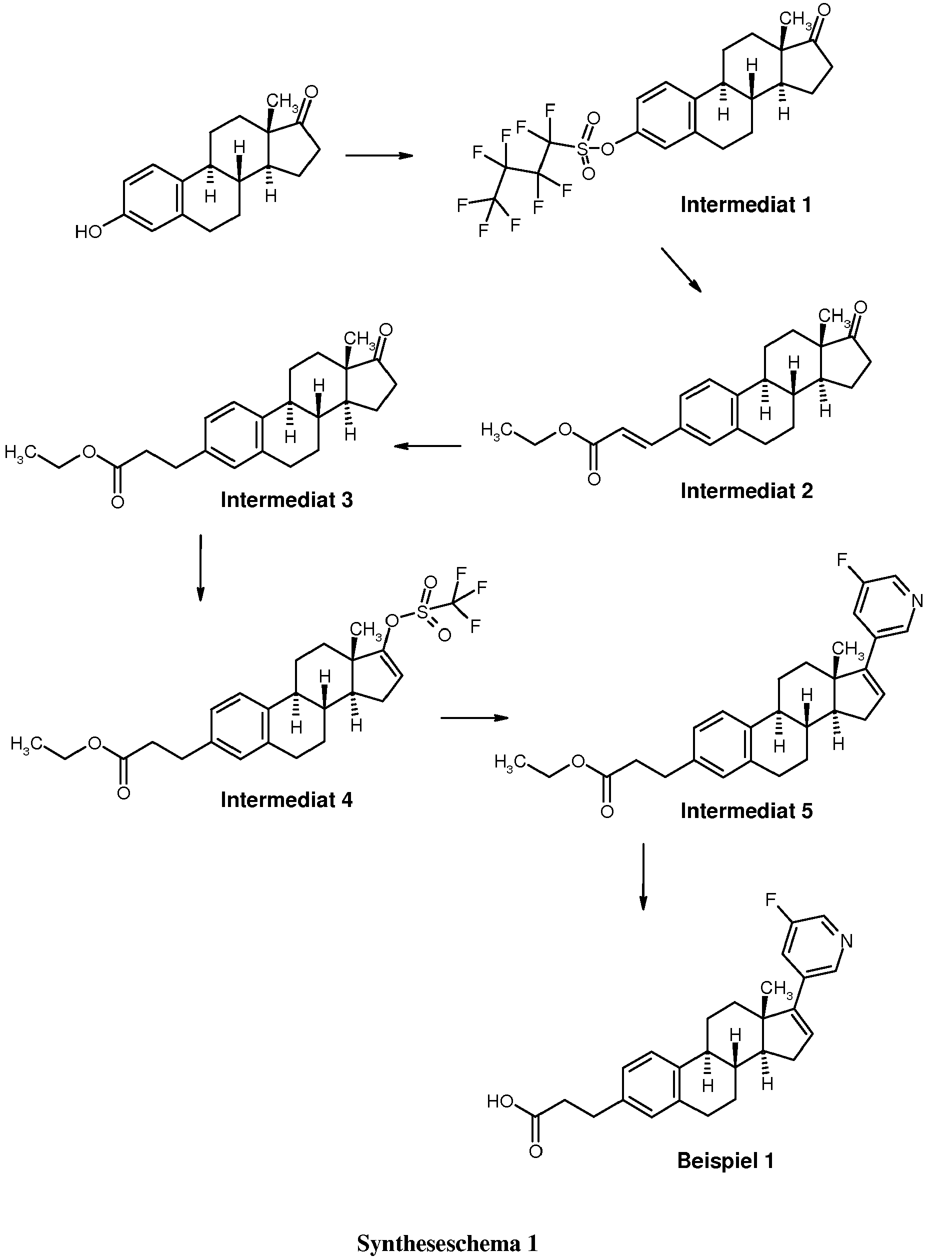

Eine weitere Teilmenge der erfindungsgemäßen Verbindungen lässt sich herstellen wie exemplarisch an der Synthese der Beispiele 2 bis 7 gezeigt (Syntheseschema 2):

Ausgehend von Estron wird Intermediat 6 durch Umsetzung mit Benzylbromacetat oder Benzylchloracetat in Gegenwart einer Base wie zum Beispiel Natriumhydrid, Kalium-*tert-*butylat, Cäsiumcarbonat oder Kaliumcarbonat in DMSO, 1-Methylpyrrolidin-2-on, DMF oder Tetrahydrofuran umgesetzt. Bevorzugt ist die Umsetzung mit Benzylbromacetat in Gegenwart von Kaliumcarbonat in Tetrahydrofuran. Das Intermediat 7 wird analog zur Herstellung von Intermediat 4 synthetisiert. Ausgehend von Intermediat 7 werden dann die Beispielverbindungen 2 bis 7 unter Verwendung von Reaktionsbedingungen wie bei der Herstellung von Intermediat 5 mittels der Suzuki-Reaktion hergestellt. Es werden bevorzugt die entsprechenden aromatischen stickstoffhaltigen Boronsäuren oder Boronsäurepinakolester verwendet. Ausgehend von den Beispielen 2,3,4,5,6 und 7 lassen sich weitere Beispielverbindungen herstellen wie exemplarisch an der Herstellung von Beispiel 19 gezeigt. Hierzu wird Beispielverbindung 2 (mit der Bedeutung A gleich 5-(Trifluormethyl)pyridin-3-yl) mit Ethylamin im Rahmen einer dem Fachmann bekannten Amidsynthese-Reaktion umgesetzt. Bevorzugt ist Verwendung von 1,1'-Carbonyldiimidazol und Imidazol Hydrochlorid in 2-Methyltetrahydrofuran wie in Orgonic Process Research & Development 2009, 13, 106-113 beschrieben.

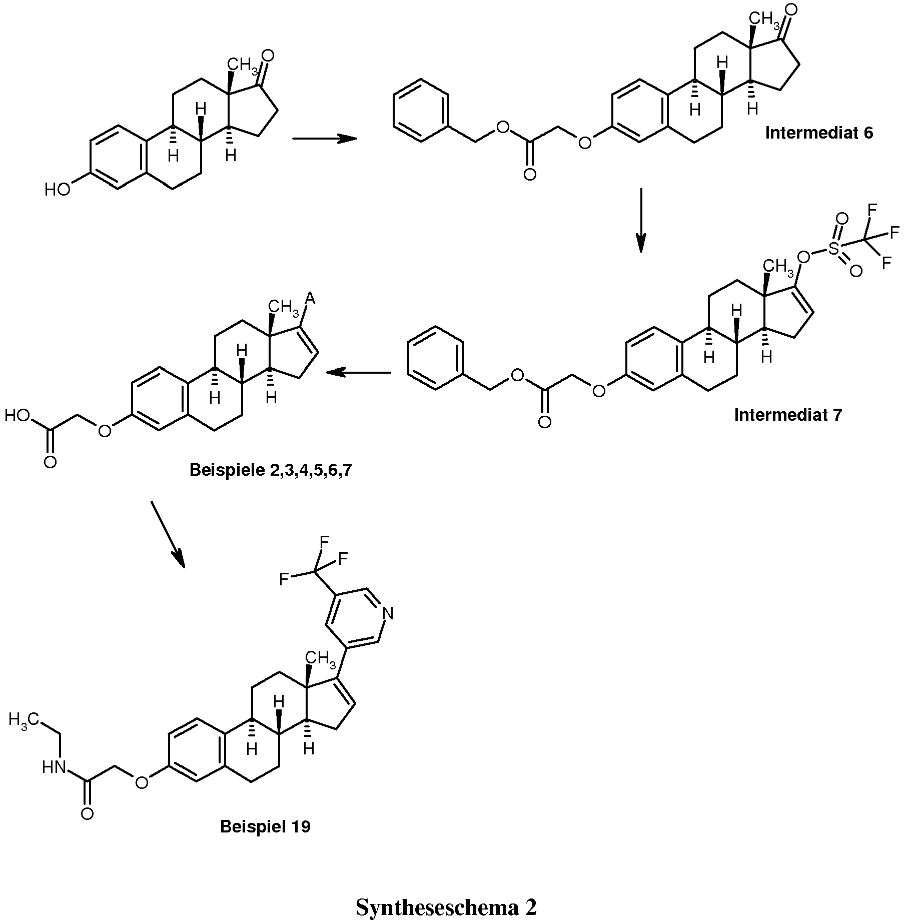

Eine weitere Teilmenge der erfindungsgemäßen Verbindungen lässt sich herstellen wie exemplarisch an der Synthese der Beispiele 8 bis 18 gezeigt (Syntheseschema 3):
3-Methoxyestra-1,3,5(10),16-tetraen-17-yltrifluormethansulfonat (S. Cacchi, E. Morera, Synthesis, 1986, 4, 320 - 322) wird analog zur Herstellung des Intermediates 5 mittels der Suzuki-Reaktion mit den entsprechenden stickstoffhaltigen aromatischen Boronsäuren oder Boronsäurepinakolestern hergestellt. Bevorzugt ist die Verwendung von 5-Fluorpyridin-3-boronsäure (für die Herstellung von Intermediat 8) oder Pyridin-3-boronsäure (für die Herstellung von Intermediat 10). Die Intermediate 9 und 11 werden durch die Umsetzung mit Bortribromid in Gegenwart von 2,6-Lutidin in Dichlormethan hergestellt. Die Herstellung der Beispielverbindungen erfolgt durch die Umsetzung mit den entsprechend substituierten Alkylhalogeniden in Gegenwart einer Base wie Natriumhydrid, Kalium-*tert*-butylat, Cäsiumcarbonat oder Kaliumcarbonat. Natriumiodid oder Kaliumiodid kann ggf. bei der Verwendung von Alkylchloriden zugegeben werden. Bevorzugt ist die Umsetzung mit Kaliumcarbonat als Base in Gegenwart von Kaliumiodid oder Natriumiodid. In dem Fall, dass der Rest R1 der Beispielverbindungen eine Carboxylgruppe enthält, werden ausgehend von den Intermediaten 9 und 11 Alkylhalogenide subsitutiert mit einer Carbonsäuremethylester oder Carbonsäureethylester-Gruppe verwendet und in einem zweiten Schritt werden die Ester durch Zugabe von Natronlauge verseift.

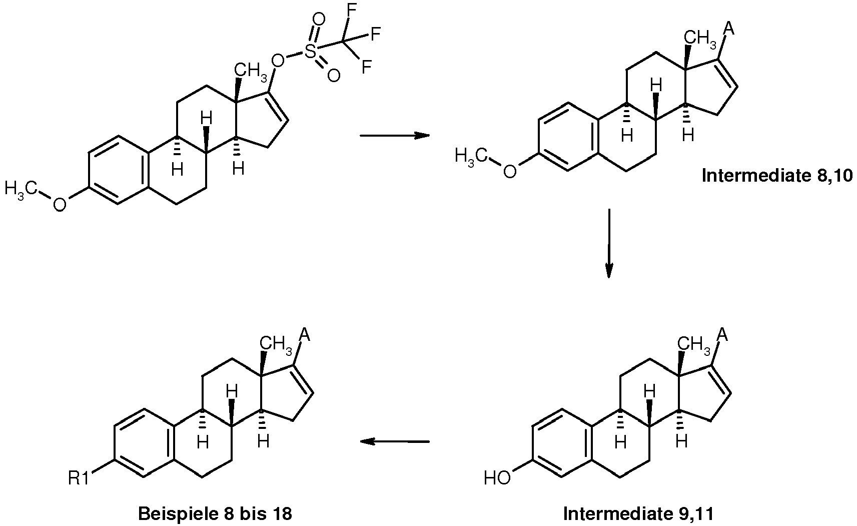

Die erfindungsgemäßen Verbindungen zeigen unvorhersehbar ein wertvolles pharmakologisches und pharmakokinetisches Wirkspektrum. Sie eignen sich daher zur Verwendung als Arzneimittel zur Behandlung und/oder Prophylaxe von Krankheiten bei Menschen und Tieren. Der Begriff "Behandlung" im Rahmen der vorliegenden Erfindung schließt die Prophylaxe ein. Die pharmazeutische Wirksamkeit der erfindungsgemäßen Verbindungen lässt sich durch ihre Wirkung als AKR1C3 Inhibitor erklären. Die erfindungsgemäßen Verbindungen sind daher besonders zur Behandlung und/oder Prophylaxe der Endometriose, von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes geeignet.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge der erfindungsgemäßen Verbindungen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe der zuvor genannten Erkrankungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: selektive Estrogen Rezeptor Modulatoren (SERMs), Estrogenrezeptor (ER) Antagonisten, Aromataseinhibitoren, 17β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen Kinasen (Mkk3/6, Mek1/2, Erk1/2), Inhibitoren der Proteinkinasen B (PKBα/β/γ; Akt1/2/3), Inhibitoren der Phosphoinositid-3-Kinasen (PI3K), Inhibitoren der Cyclinabhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten, und Nicht-steroidale Enzündungshemmer (NSAIDs).

Beispielsweise können die Verbindungen der vorliegenden Erfindung mit bekannten antihyperproliferativen, zytostatischen oder zytotoxischen Substanzen zur Behandlung von Krebserkrankungen kombiniert werden. Darüber hinaus können die erfindungsgemäßen Verbindungen auch in Kombination mit einer Strahlentherapie und/oder einer chirurgischen Intervention eingesetzt werden.

Als geeignete Kombinationswirkstoffe seien beispielhaft genannt:
131I-chTNT, Abarelix, Abiraterone, Aclarubicin, Aldesleukin, Alemtuzumab, Alitretinoin, Altretamin, Aminoglutethimid, Amrubicin, Amsacrin, Anastrozol, Arglabin, Arsentrioxidas, Asparaginase, Azacitidin, Basiliximab, BAY 80-6946, BAY 1000394, BAY 86-9766 (RDEA 119), Belotecan, Bendamustin, Bevacizumab, Bexarotene, Bicalutamid, Bisantrene, Bleomycin, Bortezomib, Buserelin, Busulfan, Cabazitaxel, Kalciumfolinat, Kalciumlevofolinat, Capecitabine, Carboplatin, Carmofur, Carmustin, Catumaxomab, Celecoxib, Celmoleukin, Cetuximab, Chlorambucil, Chlormadinon, Chlormethine, Cisplatin, Cladribin, Clodronsäure, Clofarabine, Crisantaspase, Cyclophosphamid, Cyproteron, Cytarabine, Dacarbazine, Dactinomycin, Darbepoetin alfa, Dasatinib, Daunorubicin, Decitabine, Degarelix, Denileukin diftitox, Denosumab, Deslorelin, Dibrospidiumchlorid, Docetaxel, Doxifluridin, Doxorubicin, Doxorubicin + Estron, Eculizumab, Edrecolomab, Elliptiniumazetat, Eltrombopag, Endostatin, Enocitabine, Epirubicin, Epitiostanol, Epoetin alfa, Epoetin beta, Eptaplatin, Eribulin, Erlotinib, Estradiol, Estramustine, Etoposide, Everolimus, Exemestan, Fadrozole, Filgrastim, Fludarabin, Fluorouracil, Flutamid, Formestane, Fotemustine, Fulvestrant, Galliumnitrat, Ganirelix, gefitinib, Gemcitabine, Gemtuzumab, Glutoxim, Goserelin, Histaminedihydrochlorid, Histrelin, Hydroxycarbamid, I-125 Pellets, Ibandronicsaüre, Ibritumomab Tiuxetan, Idarubicin, Ifosfamid, Imatinib, Imiquimod, Improsulfan, Interferon alfa, Interferon beta, Interferon gamma, Ipilimumab, Irinotecan, Ixabepilon, Lanreotid, lapatinib, Lenalidomid, Lenograstim, Lentinan, Letrozol, Leuprorelin, Levamisol, Lisurid, Lobaplatin, Lomustine, Lonidamin, Masoprocol, Medroxyprogesterone, Megestrol, Melphalan, Mepitiostan, Mercaptopurin, Methotrexat, Methoxsalen, Methyl Mminolevulinat, Methyltestosteron, Mifamurtid, Miltefosin, Miriplatin, Mitobronitol, Mitoguazone, Mitolactol, Mitomycin, Mitotane, Mitoxantron, Nedaplatin, Nelarabine, Nilotinib, Nilutamid, Nimotuzumab, Nimustine, Nitracrine, Ofatumumab, Omeprazol, Oprelvekin, Oxaliplatin, p53 Gentherapie, Paclitaxel, Palifermin, Palladium-103 Pellets, Pamidronicsäure, Panitumumab, Pazopanib, Pegaspargase, PEG-Epoetin beta (methoxy PEG-Epoetin beta), Pegfilgrastim, Peginterferon alfa-2b, Pemetrexed, Pentazocin, Pentostatin, Peplomycin, Perfosfamid, Picibanil, Pirarubicin, Plerixafor, Plicamycin, Poliglusam, Polyestradiolphosphat, Polysaccharide-K, Porfimer Sodium, Pralatrexat, Prednimustine, Procarbazine, Quinagolid, Radium-223 Chlorid, Raloxifen, Raltitrexed, Ranimustine, Razoxane, Regorafenib, Risedronicsäure, Rituximab, Romidepsin, Romiplostim, Sargramostim, Sipuleucel-T, Sizofiran, Sobuzoxan, Sodium Glycididazol, Sorafenib, Streptozocin, Sunitinib, Talaporfin, Tamibaroten, Tamoxifen, Tasonermin, Teceleukin, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Tioguanin, Tocilizumab, Topotecan, Toremifen, Tositumomab, Trabectedin, Trastuzumab, Treosulfan, Tretinoin, Trilostan, Triptorelin, Trofosfamid, Tryptophan, Ubenimex, Valrubicin, Vandetanib, Vapreotid, Vemurafenib, Vinblastin, Vincristin, Vindesin, Vinflunin, Vinorelbin, Vorinostat, Vorozol, Yttrium-90 Mikroglasskugeln, Zinostatin, Zinostatin Stimalamer, Zoledronicsäure, Zorubicin.

Vorzugsweise betrifft die vorliegende Erfindung Arzneimittel, enthaltend mindestens eine erfindungsgemäße Verbindung und einen oder mehrere der folgenden Wirkstoffe, insbesondere zur Behandlung und/oder Prophylaxe Androgenrezeptor-abhängiger proliferativer Erkrankungen:
LHRH (luteinizing hormone-releasing hormone) Agonisten,
LHRH (luteinizing hormone-releasing hormone) Antagonisten,
C(17,20)-Lyase-Inhibitoren,
5-α-Reduktase-Inhibitoren Typ I,
5-α-Reduktase-Inhibitoren Typ II,
gemischte 5-α-Reduktase-Inhibitoren Typ I/II,
α-Strahlung emittierende Radiopharmazeutika zur Behandlung von Knochenmetastasen, wie
z.B. Radium-223 Chlorid,
Zytostatika,
VEGF (Vascular Endothelial Growth Factor) -Kinase-Inhibitoren,
Antigestagene,
Antiestrogene,
EGF-Antikörper,
Estrogene oder
andere Androgenrezeptor Antagonisten,
Poly (ADP-ribose) Polymerase I Inhibitoren, oder
Bi-specific T-cell engagers (BiTE) gekoppelt an einem Zelloberflächenprotein wie zum Beispiel prostate-specific membrane antigen (PSMA).

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die zuvor genannten Indikationen.

Die Verbindungen können, sowohl nach oraler als auch parenteraler Gabe, für die zuvor genannten Indikationen eingesetzt werden.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate, intrauterine Spiralen, Vaginalringe oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Bei oraler Applikation beträgt die Menge pro Tag etwa 0,01 bis 100 mg/kg Körpergewicht. Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg / kg Körpergewicht je Tag betragen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Abkürzungsverzeichnis Chemie

Abkürzungen und Akronyme:

| | |
|---|---|
| DMF | *N*,*N*-Dimethylformamid |
| DMSO | Dimethylsulfoxid |
| d. Th. | der Theorie (bei Ausbeute) |
| h | Stunde(n) |
| HPLC | Hochdruck-, Hochleistungsflüssigkeitschromatographie |
| LC-MS | Flüssigkeitschromatographie-gekoppelte Massenspektroskopie |
| ES-MS | Elektrospray Massenspektrometrie |
| min | Minute(n) |
| MS | Massenspektrometrie |
| NMR | Kernresonanzspektroskopie |
| RT | Raumtemperatur |
| TFA | Trifluoressigsäure |
| THF | Tetrahydrofuran |

### Reinigung der erfindungsgemäßen Verbindungen

In einigen Fällen konnten die erfindungsgemäßen Verbindungen durch präparative HPLC zum Beispiel durch ein Autopurifier-Gerät der Firma Waters (Detektion der Verbindungen durch UV-Detektion sowie Elektrospray-Ionisation) in Kombination mit kommerziell erhältlichen, vorgepackten HPLC Säulen (zum Beispiel Säule XBridge (Firma Waters), C18, 5µm, 30 x 100mm) gereinigt werden. Als Lösemittelsystem wurde Acetonitril / Wasser mit Zusatz von Ameisensäure verwendet. Weitere, dem Fachmann bekannte Zusätze wie zum Beispiel Ammoniak, Ammoniumacetat oder Trifluoressigsäure können verwendet werden. Statt Acetonitril kann beispielsweise auch Methanol verwendet werden.

In einigen Fällen wurde folgende Methode für die präparative HPLC Trennung verwendet:

| | |
|---|---|
| *System:* | Waters Autopurificationsystem: Pump 2545, Sample Manager 2767, CFO, DAD 2996, ELSD 2424, SQD |
| *Säule:* | XBrigde C18 5µm 100x30 mm |
| *Lösemittel:* | A = H₂O + 0.1 % Vol. Ameisensäure (99%) |
| | B = Acetonitril |
| *Gradient:* | 0-1 min 1% B, 1-8 min 1-99% B, 8-10 min 99% B |
| *Fluss:* | 50 ml/min |
| *Temperatur:* | RT |
| *Injektion:* | 1 x 2.5 ml |
| *Detektion:* | DAD scan range 210-400 nm MS ESI+, ESI-, scan range 160-1000 m/z |

Zum Entfernen des HPLC-Lösemittelgemisches wurde eine Gefriertrocknung oder eine Vakuumzentrifugation verwendet. Liegen die so erhaltenen Verbindungen als TFA-Salze bzw. Formiatsalze vor, so können diese in die jeweiligen freien Basen mittels dem Fachmann bekannten Standard-Laborprozeduren überführt werden.

In einigen Fällen konnten die erfindungsgemäßen Verbindungen durch Chromatographie an Kieselgel gereinigt werden. Hierbei wurden zum Beispiel vorgepackte Silica Gel Cartridges (zum Beispiel von der Firma Separtis, *Isolute*® *Flash silica gel)* in Kombination mit dem Flashmaster II Chromatograhiegerät (Argonaut/Biotage) und Chromatographielösemittel bzw. -gemische wie zum Beispiel Hexan, Ethylacetat sowie Dichlormethan und Methanol in Betracht.

### Strukturanalytik der erfindungsgemäßen Verbindungen:

In einigen Fällen wurden die erfindungsgemäßen Verbindungen durch LC-MS analysiert:
   In einigen Fällen wurde folgende Analytikmethode verwandt:
      Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril;
      Gradient: 0-1.6 min 1-99% B, 1.6-2.0 min 99% B; Fluss 0.8 ml/min; Temperatur: 60 °C; Injektion: 2 µl; DAD scan: 210-400 nm

Bei den NMR-Daten der erfindungsgemäßen Verbindungen gelten folgende Bedeutungen:

| | |
|---|---|
| s | Singulett |
| d | Dublett |
| t | Triplett |
| q | Quartett |
| quin | Quintett |
| m | Multiplett |
| br | breit |
| mc | Zentriertes Multiplett |

### Synthese der erfindungsgemäßen Verbindungen:

### Intermediat 1

### 17-Oxoestra-1,3,5(10)-trien-3-yl-1,1,2,2,3,3,4,4,4-nonafluorbutan-1-sulfonat

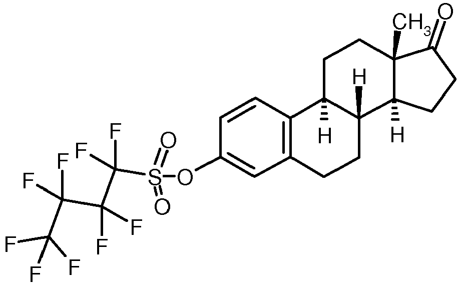

30.0 g (111 mmol) Estron und 46.0 g (222 mmol) Kaliumcarbonat wurden in 300 ml THF vorgelegt. Es wurden 40.2 g (133 mmol) 1,1,2,2,3,3,4,4,4-Nonafluorbutan-1-sulfonylfluorid zugegeben und die Mischung wurde 7 h unter Rückfluss und 18 h bei RT gerührt. Danach addierte man erneut 0.2 Äquivalente 1,1,2,2,3,3,4,4,4-butan-1-sulfonylfluorid und erhitzte 4 h unter Rückfluss. Man filtrierte den Feststoff ab und engte das Filtrat bis zur Hälfte ein, goss auf gesättigte Natriumchloridlösung und rührte 35 min. Danach wurden die Phasen getrennt und man extrahierte zweimal mit Essigester und wusch die vereinigten organischen Phasen mit Wasser und gesättigter Natriumchloridlösung und trocknete über Natriumsulfat. Man entfernte das Lösemittel und rührte aus Hexan aus. Man erhielt 63.6 g eines Feststoffes. C₂₂H₂₁F₉O₄S. ¹H-NMR (300MHz, DMSO-d₆): δ [ppm]= 0.80 (s, 3H), 1.27 - 1.64 (m, 6H), 1.66 - 1.80 (m, 1H), 1.85 - 2.12 (m, 3H), 2.18 - 2.43 (m, 3H), 2.81 - 2.94 (m, 2H), 7.11 - 7.21 (m, 2H), 7.43 (d, 1H).

### Intermediat 2

### (E)-Ethyl-3-(17-oxoestra-1,3,5(10)-trien-3-yl)acrylat

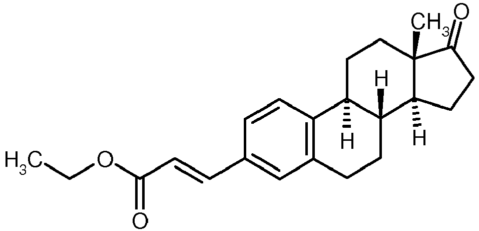

Eine Mischung aus 500 mg (0.91 mmol) 17-Oxoestra-1,3,5(10)-trien-3-yl-1,1,2,2,3,3,4,4,4-nonafluorbutan-1-sulfonat, 181 mg Acrylsäureethylester, 144 Microliter Triethylamin, 47 mg (0.15 mmol) Tri-2-tolylphosphin und 14 mg (0.06 mmol) Palladium(II)acetat in 8 ml Acetonitril wurde bei 150°C / 100 Watt in der Mikrowelle erhitzt. Man vereinigte mit zwei analogen Reaktionsansätzen (ausgehend von jeweils 1 g 17-Oxoestra-1,3,5(10)-trien-3-yl-1,1,2,2,3,3,4,4,4-nonafluorbutan-1-sulfonat). Man filtrierte über Celite, goss auf wässrige Ammoniumchloridlösung und ließ 30 min rühren. Man extrahierte dreimal mit Dichlormethan, wusch mit gesättigter Natriumchloridlösung, trocknete über Natriumsulfat und engte ein. Nach säulenchromatographischer Reinigung an Kieselgel (Hexan/Ethylacetat) wurden 287 mg eines Feststoffes erhalten. C₂₃H₂₈O₃. ¹H-NMR (400MHz, DMSO-d₆): δ [ppm]= 0.80 (s, 3H), 1.21 (t, 3H), 1.28 - 1.45 (m, 3H), 1.45 - 1.60 (m, 3H), 1.69 - 1.80 (m, 1H), 1.87 - 2.10 (m, 3H), 2.18 - 2.29 (m, 1H), 2.32 - 2.45 (m, 2H), 2.79 - 2.88 (m, 2H), 4.14 (q, 2H), 6.51 (d, 1H), 7.29 (d, 1H), 7.39 (s, 1H), 7.41 - 7.45 (m, 1H), 7.54 (d, 1H).

### Intermediat 3

### Ethyl-3-(17-oxoestra-1,3,5(10)-trien-3-yl)propanoat

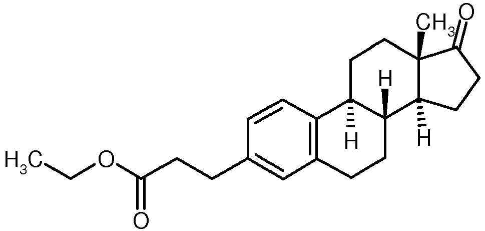

282 mg (0.80 mmol) (E)-Ethyl-3-(17-oxoestra-1,3,5(10)-trien-3-yl)acrylat in 10 ml Ethylacetat wurden mit 100 mg Pd/Kohle (10%ig) versetzt. Bei RT wurde 1 h Wasserstoff eingeleitet. Der Katalysator wurde abfiltriert und der Rückstand mit insgesamt 10 ml Ethylacetat nachgewaschen. Nach dem Entfernen des Lösemittels im Vakuum erhielt man 278 mg (98% d. Th.) Produkt. C₂₃H₃₀O₃.
1H-NMR (300MHz, Chloroform-d): δ [ppm]= 0.90 (s, 3H), 1.25 (t, 3H), 1.35 - 1.73 (m), 1.91 - 2.21 (m, 4H), 2.23 - 2.34 (m, 1H), 2.35 - 2.56 (m, 2H), 2.60 (t, 2H), 2.84 - 2.97 (m, 4H), 4.14 (q, 2H), 6.95 (s, 1H), 7.00 (d, 1H), 7.22 (d, 1H).

### Intermediat 4

### Ethyl-3-(17-{[(trifluormethyl)sulfonyl]oxy}estra-1,3,5(10),16-tetraen-3-yl)propanoat

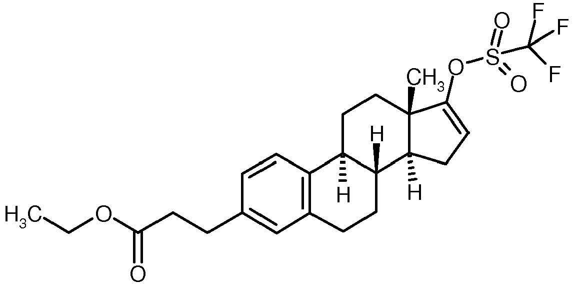

Zu einer Lösung aus 274 mg (0.77 mmol) Ethyl-3-(17-oxoestra-1,3,5(10)-trien-3-yl)propanoat in 10 ml Dichlormethan wurden 0.25 ml (1.16 mmol) 2,6-Di-*tert-*butylpyridin zugetropft. Dann addierte man 0.16 ml (0.93 mmol) Trifluormethansulfonsäureanhydrid und ließ 24 h bei RT rühren. Man goss vorsichtig auf 70 ml gesättigte Natriumhydrogencarbonatlösung, trennte die Phasen, extrahierte zweimal mit Dichlormethan, wusch die vereinigten organischen Phasen mit gesättigter Natriumhydrogencarbonatlösung, zweimal mit 1M wässriger Salzsäurelösung, Natriumchloridlösung, trocknete über Natriumsulfat und engte ein. Man erhielt 522 mg eines braunen Öles. C₂₄H₂₉F₃O₅S. ¹H-NMR (400MHz, Chloroform-d, ausgewählte Signale): δ [ppm]= 1.00 (s, 3H). 1.25 (t, 3H), 1.80 (td, 1H), 1.85 - 1.98 (m, 2H), 2.04 - 2.17 (m, 1H), 2.25 - 2.47 (m, 3H), 2.55 - 2.67 (m), 2.79 - 2.96 (m), 4.14 (q, 2H), 5.58 - 5.67 (m, 1H), 6.95 (s, 1H), 7.00 (d, 1H), 7.19 (d, 1H).

### Intermediat 5

### Ethyl-3-(17-(5-fluorpyridin-3-yl)estra-1,3,5(10),16-tetraen-3-yl)propanoat

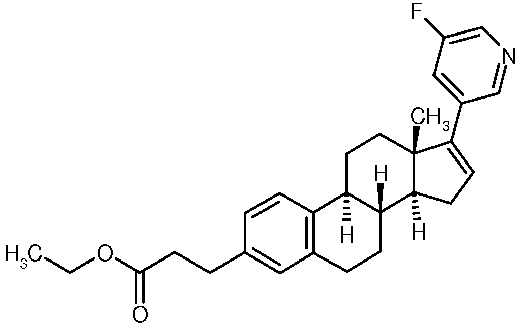

522 mg (1.07 mmol) Ethyl-3-[17-{[(trifluormethyl)sulfonyl]oxy}estra-1,3,5(10),16-tetraen-3-yl]propanoat wurden in 5 ml Toluol und 3 ml Ethanol vorgelegt. Nun gab man 212 mg (1.4 Äquivalente) 5-Fluorpyridin-3-boronsäure, 91 mg LiCl, 1.3 ml 2M wässrige Natriumcarbonatlösung und 38 mg Bis(triphenylphosphin)palladium(II)chlorid zu und erwärmte 90 min bei 120°C/300W in der Mikrowelle. Man filtrierte, trennte die Phasen und extrahierte die wässrige Phase zweimal mit je 20 ml Ethylacetat. Die vereinigten organischen Phasen wurden mit gesättigter Natriumhydrogencarbonatlösung und gesättigter Natriumchloridlösung gewaschen, über Natriumsulfat getrocknet und eingeengt. Nach säulenchromatographischer Reinigung an Kieselgel (Hexan/Ethylacetat) erhielt man 108 mg eines gelblichen Öles. C₂₈H₃₂FNO₂. ¹H-NMR (300MHz, CHLOROFORM-d): δ [ppm]= 1.04 (s, 3H), 1.25 (t), 1.2 - 1.90 (m), 1.91 - 2.04 (m, 1H), 2.08 - 2.26 (m, 2H), 2.26 - 2.51 (m, 3H), 2.61 (t, 2H), 2.82 - 2.97 (m), 4.14 (q, 2H), 6.07 - 6.12 (m, 1H), 6.91 - 7.05 (m, 2H), 7.22 (d, 1H), 7.40 (dt, 1H), 8.34 (d, 1H), 8.45 - 8.51 (m, 1H).

### Intermediat 6

### Benzyl-[(17-oxoestra-1,3,5(10)-trien-3-yl)oxy]acetat

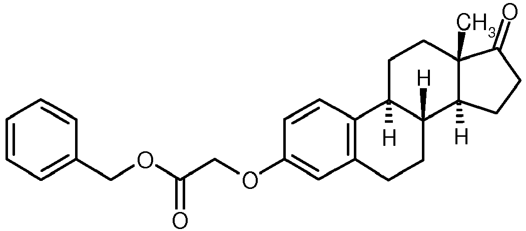

Eine Mischung aus 5.00 g (18 mmol) Estron, 3.5 ml Benzylbromacetat (22 mmol) und 7.67 g (55 mmol) Kaliumcarbonat und 70 ml THF wurde unter Rückfluss über Nacht erhitzt. Man gab nochmals 0.2 Äquivalente Benzylbromacetat hinzu und ließ weitere 3 h unter Rückfluss rühren. Das Reaktionsgemisch wurde mit Wasser versetzt. Die Phasen ließ man trennen und extrahierte die wässrige Phase zweimal mit Ethylacetat. Die vereinigten org. Phasen wurden mit Wasser, gesättigter Natriumchloridlösung gewaschen, über Natriumsulfat getrocknet, filtriert und eingeengt. Nach säulenchromatographischer Reinigung an Kieselgel (Hexan/Ethylacetat) erhielt man 7.91 g eines farblosen Öles. C₂₇H₃₀O₄. ¹H-NMR (300MHz, Chloroform-d): δ [ppm]= 0.91 (s, 3H), 1.35 - 1.73 (m), 1.90 - 2.59 (m), 2.78 - 2.96 (m, 2H), 4.64 (s, 2H), 5.24 (s, 2H), 6.60 - 6.67 (m, 1H), 6.70 (dd, 1H), 7.19 (d, 1H), 7.35 (s, 5H).

### Intermediat 7

### Benzyl-[(17-{[(trifluormethyl)sulfonyl]oxy}estra-1,3,5(10),16-tetraen-3-yl)oxy]acetat

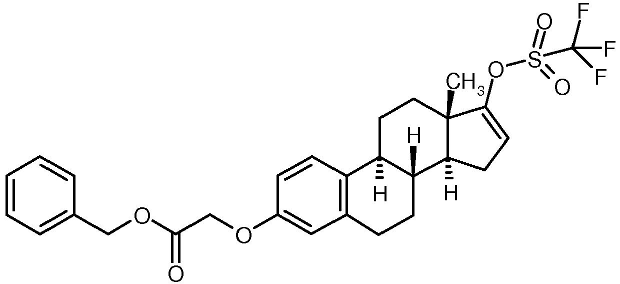

7.9 g (18.9 mmol) Benzyl-[(17-oxoestra-1,3,5(10)-trien-3-yl)oxy]acetat wurden analog zur Herstellung von Intermediat 4 zu 10.1 g der Titelverbindung als Rohprodukt umgesetzt. C₂₈H₂₉F₃O₆S. ¹H-NMR (400MHz, DMSO-d₆): δ [ppm]= 0.92 (s, 3H), 1.29 - 1.61 (m), 1.65 - 1.86 (m, 3H), 2.02 - 2.15 (m, 1H), 2.17 - 2.40 (m, 3H), 2.66 - 2.78 (m, 2H), 4.76 (s, 2H), 5.15 (s, 2H), 5.72 (d, 1H), 6.57 (d, 1H), 6.65 (dd, 1H), 7.10 (d, 1H), 7.27 - 7.41 (m, 5H).

### Intermediat 8

### 17-(5-Fluorpyridin-3-yl)-3-methoxyestra-1,3,5(10),16-tetraen

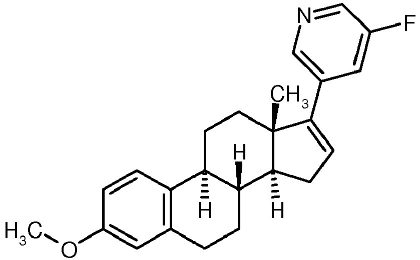

Zu einer Mischung aus 14.6 g (35.1 mmol) 3-Methoxyestra-1,3,5(10),16-tetraen-17-yltrifluormethansulfonat (S. Cacchi, E. Morera, Synthesis, 1986, 4, 320 - 322), 6.92 g (1.4 Äquiv.) 5-Fluorpyridin-3-boronsäure, 2.97 g (2.0 Äquiv.) Lithiumchlorid, 47 ml 2M wässrige Natriumcarbonatlösung, 80 ml Ethanol und 100 ml Toluol addierte man 1.23 g (0.05 Äquiv.) Bis(triphenylphosphin)palladium(II)chlorid und erwärmte 4.5 h unter Rückfluß. Man filtrierte über Celite, trennte die Phasen und extrahierte mit Ethylacetat. Die vereinigten organischen Phasen wurden mit gesättigter wässriger Natriumhydrogencarbonatlösung und Natriumchloridlösung gewaschen, über Natriumsulfat getrocknet und eingeengt. Der Rückstand wurde durch Säulenchromatographie an Kieselgel (Hexan/Ethylacetat) aufgereinigt. Man erhielt 10.4 g (81% d. Th.) eines Feststoffes. C₂₄H₂₆FNO. ¹H-NMR (300MHz, Chloroform-d): δ [ppm]= 1.05 (s, 3H), 1.40 - 1.75 (m), 1.82 (td, 1H), 1.90 - 2.03 (m, 1H), 2.03 - 2.27 (m), 2.27 - 2.57 (m), 2.84 - 3.03 (m, 2H), 3.79 (s, 3H), 5.99 - 6.18 (m, 1H), 6.66 (d, 1H), 6.73 (dd, 1H), 7.21 (d, 1H), 7.40 (dt, 1H), 8.34 (d, 1H), 8.48 (s, 1H).

### Intermediat 9

### 17-(5-Fluorpyridin-3-yl)estra-1,3,5(10),16-tetraen-3-ol

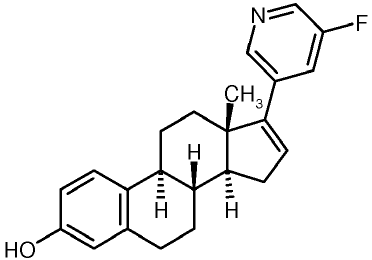

Zu 100 ml einer 1M Lösung von Bortribromid in Dichlormethan wurden 11.6 ml 2,6-Lutidin verdünnt mit 50 ml Dichlormethan in einem Temperaturbereich von 0°C bis 3°C zugetropft. Dann tropfte man 10.4 g 17-(5-Fluorpyridin-3-yl)-3-methoxyestra-1,3,5(10),16-tetraen gelöst in 50 ml Dichlormethan bei 0°C bis 3°C zu und ließ im Eisbad über Nacht auf RT kommen. Das Reaktionsgemisch wurde auf 400 ml Eiswasser gegeben und 40 min gerührt. Die Phasen wurden getrennt, dreimal mit je 50 ml Dichlormethan extrahiert und die vereinigten org. Phasen wurden mit gesättigter Natriumchloridlösung gewaschen und über Natriumsulfat getrocknet und eingeengt. Das Rohprodukt wurde bei 40°C in Hexan ausgerührt, abgesaugt und mit Hexan gewaschen. Man erhielt 13.9 g eines braunen Feststoffes (Rohprodukt). C₂₃H₂₄FNO. MS (ESIpos) gefundene Masse: 349.00.
1H-NMR (300MHz, DMSO-d6): δ [ppm]= 0.98 (s, 3H), 1.27 - 1.61 (m, 4H), 1.69 (td, 1H), 1.78 - 1.90 (m, 1H), 2.03 - 2.35 (m, 5H), 2.65 - 2.84 (m, 2H), 6.23 - 6.28 (m, 1H), 6.40 - 6.52 (m, 2H), 7.01 (d, 1H), 7.63 - 7.70 (m, 1H), 8.42 (d, 1H), 8.46 - 8.50 (m, 1H), 8.98 (s, 1H).

### Intermediat 10

### 3-Methoxy-17-(3-pyridyl)estra-1,3,5(10),16-tetraen

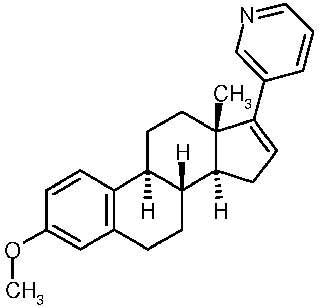

3.00 g (7.20 mmol) 3-Methoxyestra-1,3,5(10),16-tetraen-17-yltrifluormethansulfonat wurden analog zum Beispiel 8 mit 1.24 g (1.40 Äquiv.) Pyridin-3-boronsäure bei 80°C über Nacht analog umgesetzt. Man erhielt nach säulenchromatographischer Reinigung an Kieselgel (Hexan/Ethylacetat) 1.2 g der Titelverbindung als Rohprodukt. 120 mg des Rohproduktes wurden per HPLC weiter aufgereinigt. Man erhielt 60 mg der Titelverbindung. C₂₄H₂₇NO. MS (ESIpos) gefundene Masse: 345.21. ¹H-NMR (400MHz, DMSO-d₆): δ [ppm]= 0.98 (s, 3H), 1.32 - 1.61 (m, 4H), 1.71 (td, 1H), 1.83 - 1.91 (m, 1H), 2.04 - 2.14 (m, 2H), 2.17 - 2.38 (m, 3H), 2.75 - 2.89 (m, 2H), 3.66 (s, 3H), 6.11 (dd, 1H), 6.60 (d, 1H), 6.65 (dd, 1H), 7.13 (d, 1H), 7.29 - 7.34 (m, 1H), 7.76 (dt, 1H), 8.42 (dd, 1H), 8.59 (d, 1H).

### Intermediat 11

### 17-(3-Pyridyl)estra-1,3,5(10),16-tetraen-3-ol

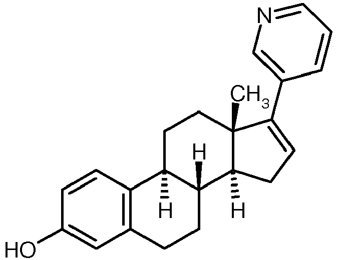

Analog zur Herstellung von Intermediat 9 wurden 1.1 g 3-Methoxy-17-(3-pyridyl)estra-1,3,5(10),16-tetraen mit Bortribromid (1M in Dichlormethan) und 2,6-Lutidin umgesetzt. Zur Aufarbeitung wurde die Mischung in Eiswasser gegossen und gerührt. Dichlormethan wurde zugegeben. Der verbleibende Feststoff wurde abgesaugt, mit Wasser und Dichlormethan gewaschen und getrocknet durch dreimalige Zugabe von Toluol und Entfernen des Toluols am Rotationsverdampfer. Nach Ausrühren aus Diethylether und Trocknen im Vakuum wurden 511 mg der Titelverbindung erhalten. C₂₃H₂₅NO. MS (ESIpos) gefundene Masse: 332.00. ¹H-NMR (400MHz, DMSO-d₆): δ [ppm]= 1.01 (s, 3H), 1.31 - 1.61 (m, 4H), 1.72 (td, 1H), 1.85 (dt, 1H), 2.09 - 2.23 (m, 3H), 2.27 - 2.41 (m, 2H), 2.66 - 2.83 (m, 2H), 6.40 - 6.45 (m, 2H), 6.49 (dd, 1H), 7.02 (d, 1H), 7.84 (dd, 1H), 8.37 - 8.42 (m, 1H), 8.67 (d, 1H), 8.82 (s, 1H).

### Herstellung der Beispielverbindungen

### Beispiel 1

### 3-[17-(5-Fluorpyridin-3-yl)estra-1,3,5(10),16-tetraen-3-yl]propansäure

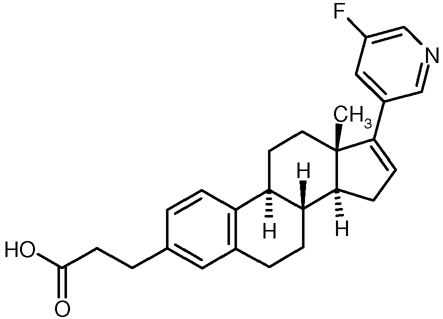

Eine Mischung aus 100 mg (0.23 mmol) Ethyl-3-[17-(5-fluorpyridin-3-yl)estra-1,3,5(10),16-tetraen-3-yl]propanoat, 3 ml THF, 0.5 ml Ethanol und 0.58 ml 2M wässriger Natronlauge wurde über Nacht bei RT gerührt, danach mit Wasser verdünnt und mit einer 10%igen wässrigen Zitronensäurelösung auf einen pH-Wert von 3 eingestellt. Die wässrige Phase wurde dreimal mit Ethylacetat extrahiert. Die vereinigten organischen Phasen wurden über Natriumsulfat getrocknet und eingeengt. Der Rückstand wurde mittels präparativer HPLC (Acetonitril/Wasser/Ameisensäure) aufgereinigt. Man erhielt 44 mg eines weißen Feststoffes. C₂₆H₂₈FNO₂. MS (ESIpos) gefundene Masse: 405.21. ¹H-NMR (300MHz, DMSO-d₆): δ [ppm]= 0.99 (s, 3H), 1.24 - 1.62 (m, 4H), 1.62 - 1.78 (m, 1H), 1.78 - 1.96 (m, 1H), 2.01 - 2.40 (m, 5H), 2.70 (t, 2H), 2.75 - 2.93 (m, 2H), 6.26 (s., 1H), 6.85 - 6.96 (m, 2H), 7.13 (d, 1H), 7.68 (d, 1H), 8.43 (d, 1H), 8.48 (s, 1H)., 12.1 (s).

### Beispiel 2

### ({17-[5-(Trifluormethyl)pyridin-3-yl]estra-1,3,5(10),16-tetraen-3-yl}oxy)essigsäure

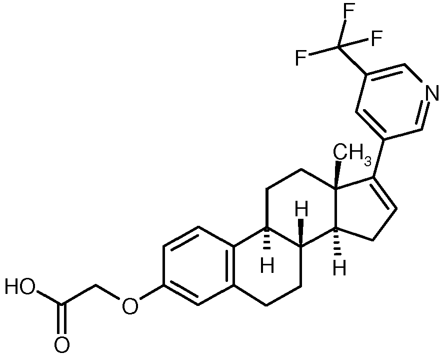

200 mg (0.36 mmol) Benzyl-{[17-{[(trifluormethyl)sulfonyl]oxy}estra-1,3,5(10),16-tetraen-3-yl]oxy}acetat wurden in 1.5 ml Toluol und 1 ml Ethanol vorgelegt. Nun gab man 97 mg (1.4 Äquivalente) 5-(Trifluormethyl)pyridin-3-boronsäure, 31 mg LiCl, 0.49 ml 2M wässrige Natriumcarbonatlösung und 12 mg 1,3-Bis(2,6-diisopropylphenyl)imidazol-2-yliden(3-chlorpyridyl)palladium(II)dichlorid (CAS 905459-27-0) zu und erwärmte 90 min bei 120°C/300W in der Mikrowelle. Man filtrierte, addierte 0.90 ml 2 M wässrige Natronlauge und erwärmte 30 min bei 120°C/300W in der Mikrowelle. Das Reaktionsgemisch wurde mit 10%iger wässriger Zitronensäurelösung auf einen pH-Wert von 3 eingestellt, man extrahierte die wässrige Phase dreimal mit Ethylacetat. Die vereinigten organischen Phasen wurden mit gesättigter Natriumchloridlösung gewaschen, über Natriumsulfat getrocknet und eingeengt. Nach Reinigung durch präparative HPLC (Acetontril/Wasser/Ameisensäure) erhielt man 43 mg eines Feststoffes. C₂₆H₂₆F₃NO₃. MS (ESIpos) gefundene Masse: 457.19. 1H-NMR (300MHz, DMSO-d6): δ [ppm]= 1.00 (s, 3H), 1.27 - 2.41 (m), 2.70 - 2.88 (m, 2H), 4.56 (s, 2H), 6.35 (s., 1H), 6.46 - 6.73 (m, 2H), 7.13 (d, 1H), 8.03 (s., 1H), 8.83 (s, 1H), 8.90 (s, 1H), 12.9 (s).

### Beispiel 3

### {[17-(5-Fluorpyridin-3-yl)estra-1,3,5(10),16-tetraen-3-yl]oxy}essigsäure

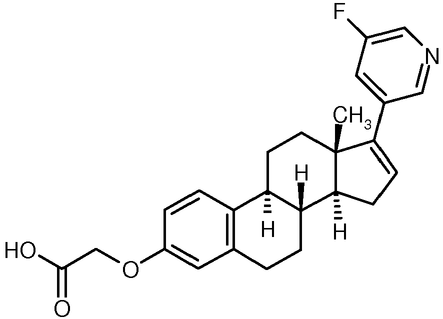

Analog zur Herstellung von Beispiel 2 wurden 200 mg (0.36 mmol) Benzyl-{[17-{[(trifluormethyl)sulfonyl]oxy}estra-1,3,5(10),16-tetraen-3-yl]oxy}acetat mit 72 mg (0.51 mmol) 5-Fluorpyridin-3-boronsäure umgesetzt. Man erhielt 31 mg (21% d. Th.) eines Feststoffes. C₂₅H₂₆FNO₃. MS (ESIpos) gefundene Masse: 407.19. 1H-NMR (300MHz, DMSO-d6): δ [ppm]= 0.98 (s, 3H), 1.26 - 1.63 (m, 4H), 1.64 - 1.78 (m, 1H), 1.79 - 1.93 (m, 1H), 2.00 - 2.39 (m, 5H), 2.73 - 2.89 (m, 2H), 4.56 (s, 2H), 6.26 (s., 1H), 6.53 - 6.66 (m, 2H), 7.13 (d, 1H), 7.68 (dd, 1H), 8.42 (d, 1H), 8.48 (s, 1H), 12.9 (s).

### Beispiel 4

### {[17-(6-Methylpyridin-3-yl)estra-1,3,5(10),16-tetraen-3-yl]oxy}essigsäure

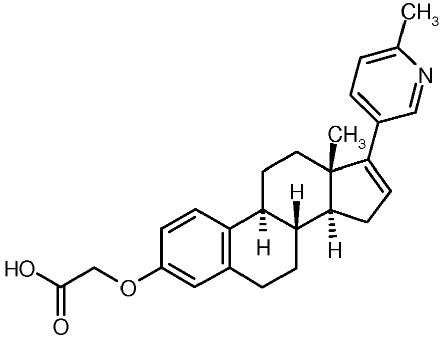

Eine Mischung aus 150 mg Benzyl-[(17-{[(trifluormethyl)sulfonyl]oxy}estra-1,3,5(10),16-tetraen-3-yl)oxy]acetat, 52 mg (1.4 Äquiv.) 2-Methyl-5-boronsäure, 23 mg (2.0 Äquiv.) Lithiumchlorid in 1.5 ml Toluol, 1 ml Ethanol und 0.37 ml 2M wässriger Natriumcarbonatlösung wurden mit 9.6 mg Bis(triphenylphosphin)palladium(II)chlorid versetzt und bei 100°C über Nacht gerührt. Man addierte 0.7 ml 2M Natronlauge und ließ bei 100°C über Nacht rühren. Das Reaktionsgemisch wurde mit Wasser verdünnt, mit Zitronensäurelösung auf pH = 4 gebracht und mit Ethylacetat dreimal extrahiert. Die vereinigten organischen Phasen wurden eingeengt und durch präparative HPLC gereinigt. Es wurden 24 mg der Titelverbindung erhalten. C₂₆H₂₉NO₃. MS (ESIpos) gefundene Masse: 403.21. ¹H-NMR (300MHz, DMSO-d₆): δ [ppm]= 0.96 (s, 3H), 1.29 - 1.62 (m, 4H), 1.69 (td, 1H), 1.80 - 1.92 (m, 1H), 1.99 - 2.39 (m, 6H), 2.41 (s, 3H), 2.71 - 2.89 (m, 2H), 4.56 (s, 2H), 6.00 - 6.07 (m, 1H), 6.56 (d, 1H), 6.59 - 6.66 (m, 1H), 7.10 - 7.22 (m, 2H), 7.61 - 7.68 (m, 1H), 8.44 (d, 1H), 12.9 (br. s., 1H).

### Beispiel 5

### {[17-(Pyrimidin-5-yl)estra-1,3,5(10),16-tetraen-3-yl]oxy}essigsäure

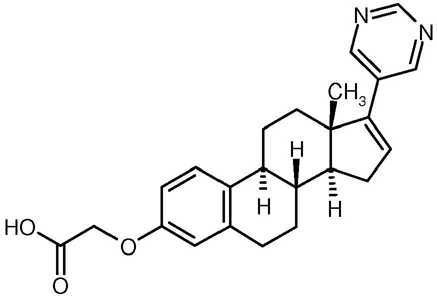

Analog zum Beispiel 4 wurden 150 mg Benzyl-[(17-{[(trifluormethyl)sulfonyl]oxy}estra-1,3,5(10),16-tetraen-3-yl)oxy]acetat mit 47 mg Pyrimidin-5-boronsäure umgesetzt. Nach Reinigung durch präparative HPLC erhielt man 42 mg der Titelverbindung. C₂₄H₂₆N₂O₃. MS (ESIpos) gefundene Masse: 390.19. ¹H-NMR (400MHz, DMSO-d₆): δ [ppm]= 0.98 (s, 3H), 1.33 - 1.62 (m, 4H), 1.72 (td, 1H), 1.83 - 1.91 (m, 1H), 2.08 - 2.16 (m, 2H), 2.18 - 2.38 (m, 3H), 2.62 - 2.65 (m, 1H), 2.73 - 2.87 (m, 2H), 4.55 (s, 2H), 6.27 - 6.31 (m, 1H), 6.57 (d, 1H), 6.63 (dd, 1H), 7.13 (d, 1H), 8.82 (s, 2H), 9.04 (s, 1H).

### Beispiel 6

### 5-[3-(Carboxymethoxy)estra-1,3,5(10),16-tetraen-17-yl]nicotinsäure

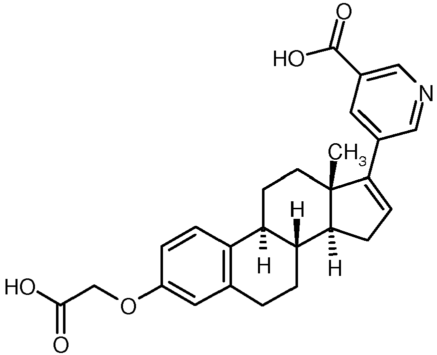

Analog zum Beispiel 4 wurden 150 mg Benzyl-[(17-{[(trifluormethyl)sulfonyl]oxy}estra-1,3,5(10),16-tetraen-3-yl)oxy]acetat mit 100 mg Methyl-5-(4,4,5,5-tetramethyl-1,3,2-dioxaborolan-2-yl)nicotinat umgesetzt. Nach Reinigung durch präparative HPLC und Ausrühren des erhaltenen Feststoffes aus Diethylether erhielt man 39 mg der Titelverbindung. C₂₆H₂₇NO₅. MS (ESIpos) gefundene Masse: 433.19. ¹H-NMR (300MHz, DMSO-d₆): δ [ppm]= 0.99 (s, 3H), 1.30 - 1.63 (m, 5H), 1.63 - 1.79 (m, 1H), 1.82 - 1.93 (m, 1H), 2.04 - 2.40 (m, 6H), 2.73 - 2.85 (m, 2H), 4.56 (s, 2H), 6.23 - 6.27 (m, 1H), 6.57 (d, 1H), 6.63 (dd, 1H), 7.13 (d, 1H), 8.16 (t, 1H), 8.81 (d, 1H), 8.91 (d, 1H), 13.2 (br. s).

### Beispiel 7

### {[17-(6-Methylpyridazin-4-yl)estra-1,3,5(10),16-tetraen-3-yl]oxy}essigsäure

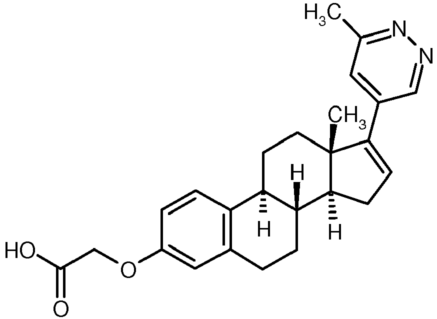

Analog zum Beispiel 4 wurden 181 mg (0.33 mmol) Benzyl-[(17-{[(trifluormethyl)sulfonyl]oxy}estra-1,3,5(10),16-tetraen-3-yl)oxy]acetat mit 101 mg (0.46 mmol) 3-Methyl-5-(4,4,5,5-tetramethyl-1,3,2-dioxaborolan-2-yl)pyridazin umgesetzt. Nach Reinigung durch präparative HPLC erhielt man ein Rohprodukt, das aus Diethylether und aus Ethylacetat ausgerührt wurde. Man erhielt 14 mg (9% d. Th.) der Titelverbindung. C₂₅H₂₈N₂O₃. MS (ESIpos) gefundene Masse: 404.21. ¹H-NMR (400MHz, DMSO-d₆): δ [ppm]= 1.01 (s, 3H), 1.32 - 1.61 (m, 4H), 1.69 (td, 1H), 1.82 - 1.90 (m, 1H), 2.08 - 2.38 (m, 5H), 2.58 (s, 3H), 2.71 - 2.88 (m, 2H), 4.51 (s, 2H), 6.54 - 6.58 (m, 2H), 6.62 (dd, 1H), 7.13 (d, 1H), 7.48 (d, 1H), 9.10 (d, 1H).

### Beispiel 8

### 3-{[17-(5-Fluorpyridin-3-yl)estra-1,3,5(10),16-tetraen-3-yl]oxy}-2,2-dimethylpropansäure

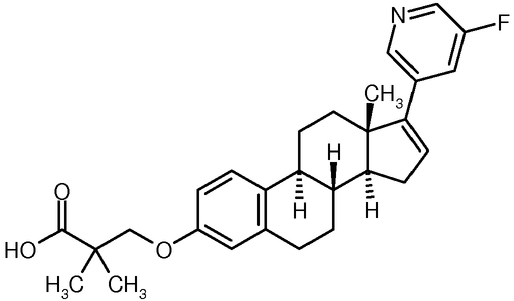

Ein Mischung aus 120 mg (0.31 mmol) 17-(5-Fluorpyridin-3-yl)estra-1,3,5(10),16-tetraen-3-ol, 61 mg (0.37 mmol, 1.2 Äquiv.) Methyl-3-chlor-2,2-dimethylpropanoat, 212 mg (1.53 mmol, 5.0 Äquiv.) Kaliumcarbonat und 5 mg Kaliumiodid in 4.6 ml DMSO wurde 18 h bei 80°C gerührt. Man addierte 0.77 ml 2M Natronlauge und ließ bei RT über Nacht rühren. Man verdünnte mit Wasser, stellte mit 10 prozentiger wässriger Zitronensäurelösung auf einen pH Wert von 3 - 4 ein, extrahierte dreimal mit Ethylacetat, engte ein und reinigte den Rückstand durch präparative HPLC. Man erhielt 7 mg eines Feststoffes. C₂₈H₃₂FNO₃. MS (ESIpos) gefundene Masse: 449.24. ¹H-NMR (400MHz, DMSO-d₆): δ [ppm]= 0.98 (s, 3H), 1.16 (s, 6H), 1.31 - 1.61 (m, 4H), 1.65 - 1.76 (m, 1H), 1.81 - 1.92 (m, 1H), 2.05 - 2.38 (m, 5H), 2.75 - 2.84 (m, 2H), 3.85 (s, 2H), 6.24 - 6.28 (m, 1H), 6.59 (d, 1H), 6.64 (dd, 1H), 7.12 (d, 1H), 7.67 (dt, 1H), 8.43 (d, 1H), 8.49 (t, 1H), 12.3 (s).

### Beispiel 9

### 4-{[17-(5-Fluorpyridin-3-yl)estra-1,3,5(10),16-tetraen-3-yl]oxy}butansäure

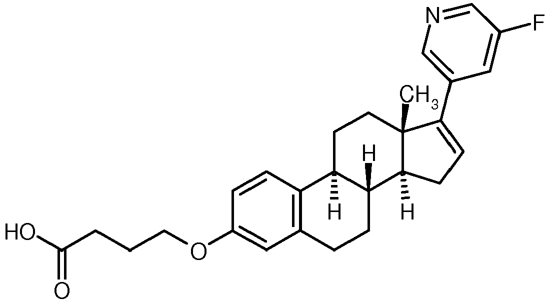

Ein Mischung aus 100 mg (0.29 mmol) 17-(5-Fluorpyridin-3-yl)estra-1,3,5(10),16-tetraen-3-ol, 78 mg (0.43 mmol, 1.2 Äquiv.) Methyl-4-brombutanoat, 198 mg (1.53 mmol, 5 .0 Äquiv.) Kaliumcarbonat und 4 mg Natriumiodid wurde bei 80°C 18 h gerührt. Danach wurden erneut 78 mg Methyl-4-brombutyrat zugegeben und über Nacht bei 120°C gerührt. Man addierte 0.72 ml 2M Natronlauge und ließ bei 40°C 3 h rühren. Man verdünnte mit Wasser, stellte mit 10%iger wässriger Zitronensäurelösung auf einen pH Wert von 4 ein, extrahierte dreimal mit Ethylacetat, engte ein und reinigte den Rückstand durch präparative HPLC. Man erhielt 43 mg eines Feststoffes. C₂₇H₃₀FNO₃. MS (ESIpos) gefundene Masse: 435.22. ¹H-NMR (400MHz, DMSO-d₆): δ [ppm]= 0.99 (s, 3H), 1.31 - 1.61 (m, 4H), 1.71 (td, 1H), 1.82 - 1.92 (m, 3H), 2.06 - 2.37 (m, 7H), 2.77 - 2.84 (m, 2H), 3.89 (t, 2H), 6.25 (dd, 1H), 6.57 - 6.81 (m, 1H), 6.62 - 6.68 (m, 1H), 7.12 (d, 1H), 7.64 - 7.70 (m, 1H), 8.43 (d, 1H), 8.48 (t, 1H), 12.1 (br. s, 1H).

### Beispiel 10

### (RS)-2-{[17-(5-Fluorpyridin-3-yl)estra-1,3,5(10),16-tetraen-3-yl]oxy}propansäure

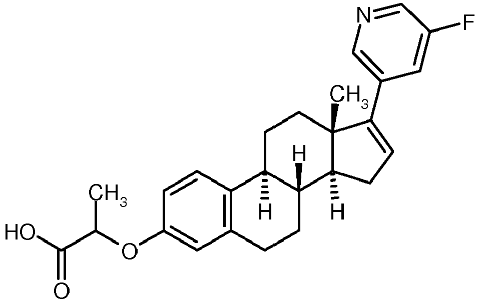

Analog zu Beispiel 8 wurden 100 mg 17-(5-Fluorpyridin-3-yl)estra-1,3,5(10),16-tetraen-3-ol mit 117 mg (3.0 Äquiv.) Ethyl-2-chlorpropanoat bei 80°C über Nacht umgesetzt. Nach Zugabe von 2M Natronlauge wurde 2.5 h bei 40°C gerührt. Man erhielt nach präparativer HPLC 44 mg der Titelverbindung. C₂₆H₂₈FNO₃. MS (ESIpos) gefundene Masse: 421.21. ¹H-NMR (300MHz, DMSO-d₆): δ [ppm]= 0.98 (s, 3H), 1.29 - 1.62 (m, 8H), 1.70 (td, 1H), 1.79 - 1.92 (m, 1H), 2.02 - 2.38 (m, 6H), 2.67 - 2.86 (m, 2H), 4.70 (q, 1H), 6.26 (br. s., 1H), 6.49 - 6.63 (m, 2H), 7.12 (d, 1H), 7.67 (dt, 1H), 8.43 (d, 1H), 8.47 - 8.50 (m, 1H), 12.9 (br. s, 1H).

### Beispiel 11

### (RS)-2-{[17-(5-Fluorpyridin-3-yl)estra-1,3,5(10),16-tetraen-3-yl]oxy}butansäure

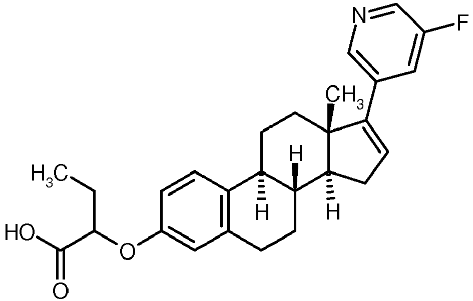

Analog zu Beispiel 8 wurden 100 mg 17-(5-Fluorpyridin-3-yl)estra-1,3,5(10),16-tetraen-3-ol mit 117 mg (3.0 Äquiv.) Methyl-2-chlorbutanoat innerhalb von 7 h bei 80°C und 72 h bei Raumtemperatur umgesetzt. Nach Zugabe von 2M Natronlauge wurde 5.5 h bei 40°C gerührt. Man erhielt nach präparativer HPLC 48 mg der Titelverbindung. C₂₇H₃₀FNO₃. MS (ESIpos) gefundene Masse: 435.22. ¹H-NMR (400MHz, DMSO-d₆): δ [ppm]= 0.95 (t, 3H), 0.99 (s, 3H), 1.31 - 1.61 (m, 4H), 1.63 - 1.92 (m, 4H), 2.09 - 2.37 (m, 5H), 2.69 - 2.89 (m, 2H), 4.48 - 4.55 (m, 1H), 6.22 - 6.28 (m, 1H), 6.52 - 6.56 (m, 1H), 6.57 - 6.63 (m, 1H), 7.12 (d, 1H), 7.64 - 7.69 (m, 1H), 8.42 (d, 1H), 8.48 (t, 1H), 12.87 (br. s., 1H).

### Beispiel 12

### (RS)-2-{[17-(5-Fluorpyridin-3-yl)estra-1,3,5(10),16-tetraen-3-yl]oxy}-3-methoxypropansäure

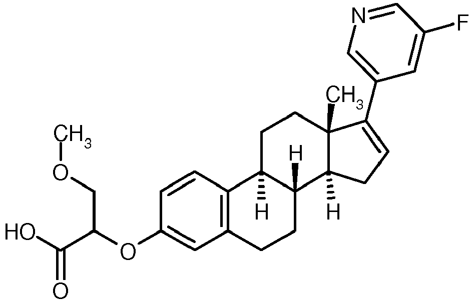

Analog zu Beispiel 8 wurden 100 mg 17-(5-Fluorpyridin-3-yl)estra-1,3,5(10),16-tetraen-3-ol mit 169 mg (3.0 Äquiv.) Methyl-2-brom-3-methoxypropanoat innerhalb von 4 h bei 80°C umgesetzt. Nach Zugabe von 2M Natronlauge wurde 5.5 h bei 40°C gerührt. Man erhielt nach präparativer HPLC 57 mg der Titelverbindung. C₂₇H₃₀FNO₄. MS (ESIpos) gefundene Masse: 451.22. ¹H-NMR (400MHz, DMSO-d₆): δ [ppm]= 0.99 (s, 3H), 1.32 - 1.61 (m, 4H), 1.71 (td, 1H), 1.81 - 1.92 (m, 1H), 2.05 - 2.37 (m, 5H), 2.71 - 2.87 (m, 2H), 3.28 (s, 3H), 3.66 - 3.78 (m, 2H), 4.79 - 4.84 (m, 1H), 6.24 - 6.27 (m, 1H), 6.54 - 6.59 (m, 1H), 6.62 (dt, 1H), 7.12 (d, 1H), 7.67 (dt, 1H), 8.42 (d, 1H), 8.48 (t, 1H), 13.01 (br. s., 1H).

### Beispiel 13

### (RS)-2-{[17-(3-Pyridyl)estra-1,3,5(10),16-tetraen-3-yl]oxy}propansäure

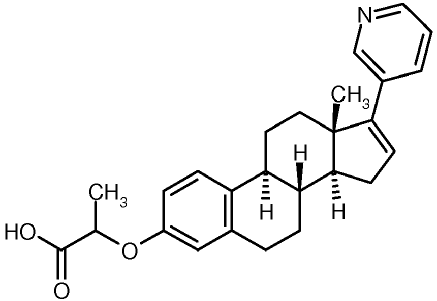

Analog zu Beispiel 8 wurden 100 mg 17-(3-Pyridyl)estra-1,3,5(10),16-tetraen-3-ol mit 123 mg (3.0 Äquiv.) Ethyl-2-chlorpropanoat bei 80°C über Nacht umgesetzt. Nach Zugabe von 2M Natronlauge wurde 4 h bei 40°C gerührt. Man erhielt nach präparativer HPLC 19 mg der Titelverbindung. C₂₆H₂₉NO₃. MS (ESIpos) gefundene Masse: 403.21. ¹H-NMR (400MHz, DMSO-d₆): δ [ppm]= 0.98 (s, 3H), 1.31 - 1.61 (m, 7H, enthält Dublett bei 1.43 ppm), 1.71 (td, 1H), 1.81 - 1.92 (m, 1H), 2.02 - 2.15 (m, 2H), 2.15 - 2.38 (m, 3H), 2.69 - 2.89 (m, 2H), 4.70 (qd, 1H), 6.11 (dd, 1H), 6.51 - 6.55 (m, 1H), 6.59 (dt, 1H), 7.12 (d, 1H), 7.29 - 7.34 (m, 1H), 7.76 (dt, 1H), 8.41 (dd, 1H), 8.58 (d, 1H), 12.9 (br. s., 1H).

### Beispiel 14

### (RS)-2-{[17-(5-Fluorpyridin-3-yl)estra-1,3,5(10),16-tetraen-3-yl]oxy}propanamid

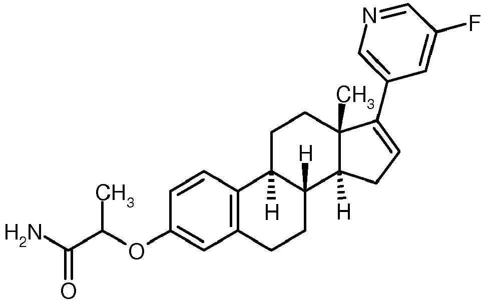

Ein Mischung aus 100 mg 17-(5-Fluorpyridin-3-yl)estra-1,3,5(10),16-tetraen-3-ol, 92 mg (3.0 Äquiv.) 2-Chlorpropanamid, 198 mg (5 Äquiv.) Kaliumcarbonat und 4 mg Natriumiodid in 3 ml DMSO wurde bei 80°C 18 h, dann bei 100°C 4 h und bei 120°C 18 h gerührt. Man verdünnte mit Wasser, extrahierte dreimal mit Ethylacetat, trocknete die vereinigten organischen Phasen über Natriumsulfat und engte ein. Nach Reinigung des Rückstandes durch präparative HPLC erhielt man 18 mg der Titelverbindung.

Aufreinigungsmethode präparative HPLC:

| | | | | |
|---|---|---|---|---|
| *System:* | Waters Autopurificationsystem: Pump 254, Sample Manager 2767, CFO, DAD 2996, ELSD 2424, SQD 3100 | | | |
| *Säule:* | XBrigde C18 5µm 100x30 mm | | | |
| *Solvent:* | A = Wasser + 0.1% Vol. HCOOH (99%) | | | |
| | B = Acetonitril | | | |
| *Gradient:* | 0-8 min 50-90% B | | | |
| *Fluss:* | 50 mL/min | | | |
| *Temperatur:* | RT | | | |
| *Detektion:* | DAD scan range 210-400 nm | | | |
| | MS ESI+, ESI-, scan range 160-1000 m/z | | | |
| | ELSD | | | |
| Fraktionen | Rt in min | Reinheit in % | Menge in mg | Peakzuordnung |
| Beispiel 14 | 4.4 - 4.7 | 98.6 | 18 | 8 -1.41min |
| *Aufarbeitung:* | Die Fraktionen wurden eingedampft, mit tBuOH versetzt, bei -65°C tiefgekühlt und abschließend gefriergetrocknet. | | | |

C₂₆H₂₉FN₂O₂. MS (ESIpos) gefundene Masse: 420.22. ¹H-NMR (400MHz, DMSO-d₆): δ [ppm]= 0.99 (s, 3H), 1.31 - 1.61 (m, 7H, enthält Dublett bei 1.37 ppm), 1.71 (td, 1H), 1.87 (dt, 1H), 2.06 - 2.38 (m, 5H), 2.70 - 2.89 (m, 2H), 4.51 (q, 1H), 6.24 - 6.27 (m, 1H), 6.58 (t, 1H), 6.64 (dt, 1H), 7.10 - 7.18 (m, 2H), 7.36 (d, 1H), 7.63 - 7.71 (m, 1H), 8.43 (d, 1H), 8.46 - 8.51 (m, 1H).

### Beispiel 15

### 3-{[17-(5-Fluorpyridin-3-yl)estra-1,3,5(10),16-tetraen-3-yl]oxy}propan-1-ol

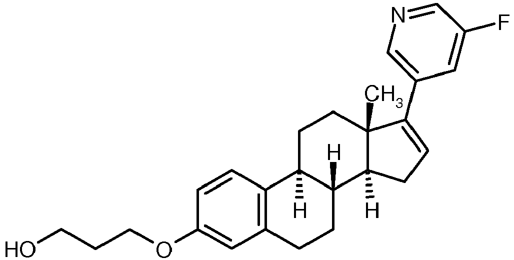

Ein Mischung aus 100 mg 17-(5-Fluorpyridin-3-yl)estra-1,3,5(10),16-tetraen-3-ol, 119 mg (3.0 Äquiv.) 3-Brompropan-1-ol, 198 mg (5 Äquiv.) Kaliumcarbonat und 4 mg Natriumiodid in 3 ml DMSO wurde bei 80°C 18 h, dann bei 100°C 4 h gerührt. Man verdünnte mit Wasser, extrahierte dreimal mit Ethylacetat, trocknete die vereinigten organischen Phasen über Natriumsulfat und engte ein. Nach Reinigung des Rückstandes durch präparative HPLC erhielt man 42 mg der Titelverbindung. C₂₆H₃₀FNO₂. MS (ESIpos) gefundene Masse: 407.23. ¹H-NMR (300MHz, DMSO-d₆): δ [ppm]= 0.98 (s, 3H), 1.29 - 1.62 (m, 4H), 1.64 - 1.91 (m, 4H), 2.03 - 2.39 (m, 5H), 2.71 - 2.90 (m, 2H), 3.44 - 3.55 (m, 2H), 3.93 (t, 2H), 4.48 (t, 1H), 6.23 - 6.28 (m, 1H), 6.59 (d, 1H), 6.64 (dd, 1H), 7.11 (d, 1H), 7.67 (dt, 1H), 8.43 (d, 1H), 8.46 - 8.51 (m, 1H).

### Beispiel 16

### 3-{[17-(5-Fluorpyridin-3-yl)estra-1,3,5(10),16-tetraen-3-yl]oxy}-2,2-dimethylpropan-1-ol

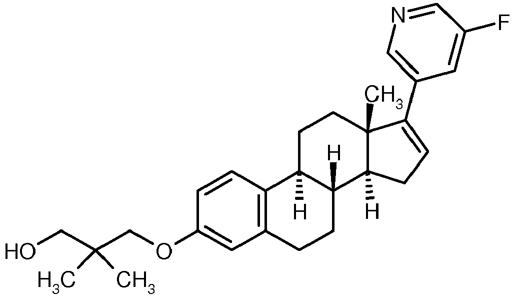

Eine Mischung aus 100 mg 17-(5-Fluorpyridin-3-yl)estra-1,3,5(10),16-tetraen-3-ol, 143 mg (3.0 Äquiv.) 3-Brom-2,2-dimethylpropan-1-ol, 198 mg (5 Äquiv.) Kaliumcarbonat und 4 mg Natriumiodid in 3 ml DMSO wurde bei 80°C 18 h, dann bei 100°C 4 h, bei 120°C 18 h und bei 150°C 5 h gerührt. Man verdünnte mit Wasser, extrahierte dreimal mit Ethylacetat, trocknete die vereinigten organischen Phasen über Natriumsulfat und engte ein. Nach Reinigung des Rückstandes durch präparative HPLC erhielt man 42 mg der Titelverbindung. C₂₈H₃₄FNO₂. MS (ESIpos) gefundene Masse: 435.26. ¹H-NMR (400MHz, DMSO-d₆): δ [ppm]= 0.87 (s, 6H), 0.99 (s, 3H), 1.31 - 1.61 (m, 4H), 1.71 (td, 1H), 1.83 - 1.91 (m, 1H), 2.06 - 2.37 (m, 5H), 2.73 - 2.87 (m, 2H), 3.23 (d, 2H), 3.60 (s, 2H), 4.52 (t, 1H), 6.26 (dd, 1H), 6.59 (d, 1H), 6.64 (dd, 1H), 7.11 (d, 1H), 7.64 - 7.70 (m, 1H), 8.43 (d, 1H), 8.48 (t, 1H).

### Beispiel 17

### (RS)-3-{[17-(5-Fluorpyridin-3-yl)estra-1,3,5(10),16-tetraen-3-yl]oxy}propan-1,2-diol

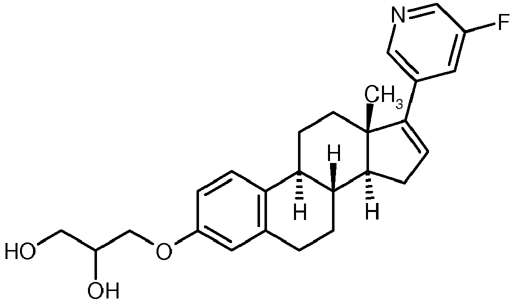

Eine Mischung aus 150 mg 17-(5-Fluorpyridin-3-yl)estra-1,3,5(10),16-tetraen-3-ol, 143 mg (3.0 Äquiv.) 3-Brom-2,2-dimethylpropan-1-ol, 296 mg (5 Äquiv.) Kaliumcarbonat und 7 mg Kaliumiodid in 3 ml DMSO wurde bei 120°C 18 h gerührt. Man verdünnte mit Wasser, extrahierte dreimal mit Ethylacetat, trocknete die vereinigten organischen Phasen über Natriumsulfat und engte ein. Nach Reinigung des Rückstandes durch präparative HPLC erhielt man 26 mg der Titelverbindung. C₂₆H₃₀FNO₃. MS (ESIpos) gefundene Masse: 435.26. ¹H-NMR (300MHz, DMSO-d₆): δ [ppm]= 0.99 (s, 3H), 1.30 - 1.62 (m, 4H), 1.71 (td, 1H), 1.81 - 1.93 (d, 1H), 2.02 - 2.39 (m, 5H), 2.74 - 2.86 (m, 2H), 3.39 (t, 2H), 3.67 - 3.80 (m, 2H), 3.90 (dd, 1H), 4.57 (t, 1H), 4.83 (d, 1H), 6.26 (br. s., 1H), 6.55 - 6.71 (m, 2H), 7.12 (d, 1H), 7.67 (dt, 1H), 8.40 - 8.52 (m, 2H).

### Beispiel 18

### (RS)-{[17-(5-Fluorpyridin-3-yl)estra-1,3,5(10),16-tetraen-3-yl]oxy}(phenyl)essigsäure

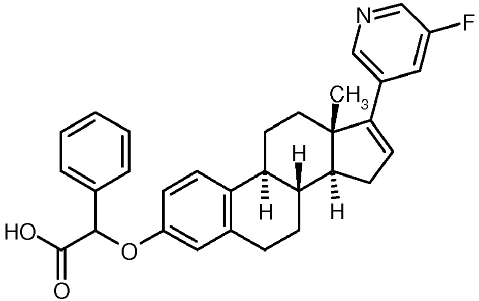

Eine Mischung aus 100 mg 17-(5-Fluorpyridin-3-yl)estra-1,3,5(10),16-tetraen-3-ol, 170 mg Ethylchlor(phenyl)acetat (3 Äquiv.), 197 mg (5 Äquiv.) Kaliumcarbonat und 4 mg Natriumiodid in 3 ml DMSO wurde bei 80°C 3 Tage gerührt. Danach addierte man 0.7 ml 2M Natronlauge und rührte 5.5 h bei 40°C. Die Reaktionsmischung wurde mit Wasser verdünnt, mit 10 prozentiger Zitronensäurelösung auf pH = 4 angesäuert und dreimal mit Ethylacetat extrahiert. Die vereinigten organischen Phasen wurden getrocknet und eingeengt. Nach Reinigung durch präparative HPLC wurden 19 mg der Titelverbindung erhalten.

Aufreinigungsmethode präparative HPLC:

| | | | | | |
|---|---|---|---|---|---|
| *System:* | Agilent: Prep 1200, 2xPrep Pump, DLA, MWD, Prep FC, | | | | |
| *Säule:* | XBrigde C18 5µm 100x30 mm | | | | |
| *Solvent:* | A = Wasser + 0.1 % TFA | | | | |
| | B = Acetonitril | | | | |
| *Gradient:* | 0-17.5 min 65-100% B, 17.5-20 min 100% B | | | | |
| *Fluss:* | 38 ml/min | | | | |
| *Temperatur:* | RT | | | | |
| *Detektion:* | UV 254 nm | | | | |
| *Fraktionen:* | | | | | |
| Beispiel 18 | | 6.1 - 6.7 min | 96 % | 19 mg | Peak 12,2 min |
| *Aufarbeitung:* | Die Fraktionen wurden eingedampft und abschließend gefriergetrocknet. | | | | |

C₃₁H₃₀FNO₃.MS (ESIpos) gefundene Masse: 483.22. 1H-NMR (300MHz, DMSO-d6): δ [ppm]= 0.98 (s, 3H), 1.30 - 1.63 (m, 4H), 1.70 (td, 1H), 1.81 - 1.91 (m, 1H), 2.04 - 2.38 (m, 5H), 2.74 - 2.84 (m, 2H), 5.71 (s, 1H), 6.23 - 6.27 (m, 1H), 6.63 - 6.73 (m, 2H), 7.14 (d, 1H), 7.32 - 7.42 (m, 3H), 7.48 - 7.54 (m, 2H), 7.64 - 7.70 (m, 1H), 8.42 (d, 1H), 8.48 (s, 1H), 13.1 (br. s., 1H).

### Beispiel 19

### N-Ethyl-2-({17-[5-(trilluormethyl)pyridin-3-yl]estra-1,3,5(10),16-tetraen-3-yl}oxy)acetamid

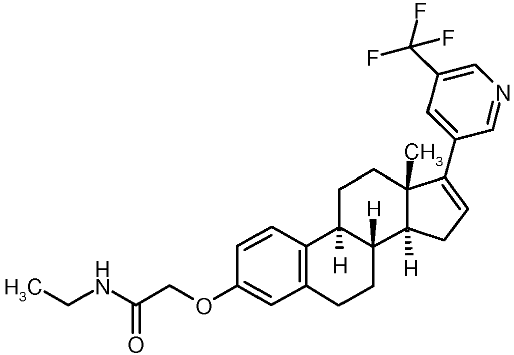

62 mg (0.14 mmol) ({17-[5-(Trifluormethyl)pyridin-3-yl]estra-1,3,5(10),16-tetraen-3-yl}oxy)essigsäure in 3 ml 2-Methyltetrahydrofuran wurden mit 66 mg (3 Äquiv.) 1,1'-Carbonyldiimidazol und 7 mg Imidazol Hydrochlorid versetzt und die Mischung wurde über Nacht bei Raumtemperatur gerührt. Man addierte 0.5 ml DMF und erwärmte 6 h auf 50°C. Man addierte 0.34 ml einer 2M Ethylamin-Lösung in THF und 57 Microliter Triethylamin und rührte 3 Tage bei Raumtemperatur. Das Reaktionsgemisch wurde mit Wasser verdünnt, dreimal mit Ethylacetat extrahiert, die vereinigten organischen Phasen wurden eingeengt und der Rückstand durch präparative HPLC gereinigt. Man erhielt 41 mg der Titelverbindung. C₂₈H₃₁F₃N₂O₂. MS (ESIpos) gefundene Masse: 484.23. ¹H-NMR (300MHz, DMSO-d₆): δ [ppm]= 0.93 - 1.07 (m, 6H), 1.33 - 1.66 (m, 4H), 1.73 (td, 1H), 1.83 - 1.92 (m, 1H), 2.05 - 2.41 (m, 5H), 2.76 - 2.86 (m, 2H), 3.06 - 3.16 (m, 2H), 4.34 (s, 2H), 6.32 - 6.38 (m, 1H), 6.62 - 6.72 (m, 2H), 7.15 (d, 1H), 7.97 - 8.06 (m, 2H), 8.81 - 8.85 (m, 1H), 8.88 - 8.92 (m, 1H).

### Pharmakologische Untersuchung der erfindungsgemäßen Verbindungen in vitro

### Beispiel 20 (AKR1C3-inhibitorische Wirkung)

Die AKR1C3-inhibitorische Wirkung der Substanzen dieser Erfindung wurde in dem in den folgenden Absätzen beschriebenen AKR1C3-Assay gemessen.

Im Wesentlichen wird die Enzymaktivität durch Quantifizierung des gebildeten Coumberols aus Coumberon gemessen (Halim, M., Yee, D.J., and Sames, D., J. AM. CHEM. SOC. 130, 14123-14128 (2008) und Yee, D.J., Balsanek, V., Bauman, D.R., Penning, T.M., and Sames, D., Proc. Natl. Acad. Sci. USA 103, 13304 - 13309 (2006)). Bei diesem Versuch kann die Zunahme des stark fluoreszierenden Coumberols durch NADPH (Nicotinamid-Adenin-Dinukleotidphosphat)-abhängige Reduktion des nicht fluoreszierenden Coumberons durch AKR1C3 bestimmt werden.

Als Enzym wurde rekombinantes humanes AKR1C3 (Aldo-keto reductase family 1 member C3) (GenBank Accession No. NM_003739) verwendet. Dieses wurde als GST (Glutathion-S-Transferase)-Fusionsprotein in *E.coli* exprimiert und mittels Gluthation-Sepharose-Affinitätschromatographie gereinigt. Durch Thrombinverdau mit anschließender Größenausschlusschromatographie wurde das GST entfernt (Dufort, I., Rheault, P., Huang, XF., Soucy, P., and Luu-The, V.,Endocrinology 140, 568-574 (1999)).

Für den Assay wurden 50 nl einer 100-fach konzentrierten Lösung der Testsubstanz in DMSO in eine schwarze low-volume 384well-Mikrotiterplatte (Greiner Bio-One, Frickenhausen, Deutschland) pipettiert, 2,0 µl einer Lösung von AKR1C3 in Assaypuffer [50 mM Kalium-Phosphatpuffer pH 7, 1 mM DTT, 0,0022% (w/v) Pluronic F-127, 0,01% BSA (w/v) und Proteaseinhibitor-Cocktail (Complete, EDTA-free Protease Inhibitor Cocktail von Roche)] hinzugegeben und die Mischung für 15 min inkubiert, um eine Vorbindung der Substanzen an das Enzym vor der Enzymreaktion zu ermöglichen. Dann wurde die Enzymreaktion durch Zugabe von 3 µl einer Lösung von NADPH (16,7 µM → Endkonzentration in 5 µl Assayvolumen ist 10 µM) und Coumberon (0,5 µM → Endkonzentration in 5 µl Assayvolumen ist 0,3 µM) in Assaypuffer gestartet und die resultierende Mischung für die Reaktionszeit von 90 min bei 22°C inkubiert. Die Konzentration der AKR1C3 wurde an die jeweilige Aktivität des Enzym-Präparats angepasst und so eingestellt, dass der Assay im linearen Bereich arbeitete. Typische Konzentrationen lagen im Bereich von 1 nM. Die Reaktion wurde durch Zugabe von 5 µl einer Stopplösung bestehend aus dem Inhibitor EM-1404 [F. Labrie et al. US Patent 6,541,463, 2003] (2 µM → Endkonzentration in 5 µM Assayvolumen ist 1 µM) gestoppt. Anschließend wurde die Fluoreszenz des Coumberols bei 520 nm (Anregung bei 380 nm) mit einem geeigneten Messgerät (Pherastar von BMG Labtechnologies) gemessen. Die Intensität der Fluoreszenz wurde als Maß für die Menge des gebildeten Coumberols und damit für die Enzymaktivität von AKR1C3 verwendet. Die Daten wurden normalisiert (Enzymreaktion ohne Inhibitor = 0 % Inhibition; alle anderen Assaykomponenten, aber kein Enzym = 100 % Inhibition). Üblicherweise wurden die Testsubstanzen auf derselben Mikrotiterplatte bei 11 verschiedenen Konzentrationen im Bereich von 20 µM bis 96,8 pM (20 µM, 5,9 µM, 1,7 µM, 0,5 µM, 0,15 µM, 44 nM, 12,9 nM, 3,8 nM, 1,1 nM, 0,3 nM und 96,8 pM, die Verdünnungsreihen wurden vor dem Assay auf der Ebene der 100-fach konzentrierten Lösung durch serielle 1:3 Verdünnungen mit 100% DMSO hergestellt) in Doppelwerten für jede Konzentration getestet, und IC₅₀-Werte wurden mit einem 4-Parameter-Fit kalkuliert.

Wie beschrieben wurden die beanspruchten pharmakologischen Substanzen auf ihre inhibitorische Wirkung auf das AKR1C3 Enzym untersucht (siehe Tabelle 1). Die beanspruchten Verbindungen zeigen eine starke Inhibition von AKR1C3 *in vitro* (IC₅₀-Werte < 200 nM) und überwiegend sogar IC₅₀-Werte < 50 nM.

**Tabelle 1: Inhibition von AKR1C3 der erfindungsgemäßen Verbindungen (angegeben sind für einen Teil der Verbindungen die Werte für zwei experimentelle Bestimmungen)**

| Beispielverbindung | AKR1C3 Enzym Inhibition IC₅₀ [nmol/l] | Beispielverbindung | AKR1C3 Enzym Inhibition IC₅₀ [nmol/l] | Beispielverbindung | AKR1C3 Enzym Inhibition IC₅₀ [nmol/l] |
|---|---|---|---|---|---|
| 1 | 7.6 | 6 | 9.2 | 13 | 2.8 |
| 1 | 8.0 | 7 | 1.4 | 14 | 20 |
| 2 | 33 | 8 | 12 | 15 | 54 |
| 2 | 23 | 8 | 11 | 16 | 124 |
| 3 | 16 | 9 | 42 | 17 | 25 |
| 3 | 8.6 | 10 | 2.7 | 18 | 10 |
| 4 | 136 | 11 | 2.5 | 19 | 53 |
| 5 | 89 | 12 | 4.0 | | |

### Beispiel 21 (Test auf AKR1C3 Inhibition im zellbasierten System)

Die Inhibierung der AKR1C3 durch die in dieser Erfindung beschriebenen Substanzen wurde in einem zellbasierten Assay unter Verwendung von Coumberol als Substrat für die AKR1C3 (Halim, M., Yee, D.J., and Sames, D., J. AM. CHEM. SOC. 130, 14123-14128 (2008) und Yee, D.J., Balsanek, V., Bauman, D.R., Penning, T.M., and Sames, D., Proc. Natl. Acad. Sci. USA 103, 13304 - 13309 (2006)) gemessen (vgl. Beispiel 20).

Als Zellsystem wurden HEK293 Zellen (ATCC, USA) verwendet (Zellkulturmedium: DMEM, 1.5 g Glukose, 10% FCS, PSG). Die Zellen wurden mit einem AKR1C3 Expressionsplasmid (pCMV6-AC-AKR1C3, GenBank Accession No. NM_003739.4) über Nacht transfiziert (X-tremeGENE HP, Roche). Am nächsten Morgen wurden die Zellen in schwarzen 96well Zellkulturplatten mit einer Zelldichte von 40000 Zellen/well ausgesät (Greiner Bio-One, Frickenhausen, Deutschland). 7 h später wurden die Zellen mit den Testsubstanzen (gelöst in 100x Konzentration in DMSO, Endkonzentration zwischen 10⁻¹¹M und 10⁻⁵M) und Coumberol (gelöst in Zellkulturmedium, Endkonzentration 5x10⁻⁶M) über Nacht inkubiert. Am folgenden Morgen wurde die Fluoreszenz des Coumberols bei 535 nm (Anregung bei 355 nm) mit einem geeigneten Messgerät (Mithras, Fa. Berthold) gemessen. Die Intensität der Fluoreszenz wurde als Maß für die Menge des gebildeten Coumberols und damit für die Enzymaktivität von AKR1C3 verwendet. Die Daten wurden normalisiert (transfizierte Zellen ohne Inhibitor, nur DMSO = 0 % Inhibition; transfizierte Zellen, 10 µM Inhibitor EM-1404 [F. Labrie et al. US Patent 6,541,463, 2003] = 100 % Inhibition) und IC₅₀-Werte wurden mit einem 4-Parameter-Fit kalkuliert.

Die beanspruchten pharmakologischen Substanzen wurden mittels des oben beschriebenen zellbasierten Assays auf ihre inhibitorische Wirkung auf das AKR1C3 Enzym untersucht (siehe Tabelle 2). Die Verbindungen zeigten eine starke Inhibition der zellulären AKR1C3 in vitro (IC₅₀-Werte < 300 nM) und überwiegend sogar IC₅₀-Werte < 100 nM.

**Tabelle 2: Inhibition von AKR1C3 der erfindungsgemäßen Verbindungen (angegeben sind für einen Teil der Verbindungen die Werte für unabhängige experimentelle Bestimmungen)**

| Beispielverbindung | Zelluläre AKR1 C3 Inhibition IC50 [nmol/l]) |
|---|---|
| 1 | 40 |
| 1 | 52 |
| 1 | 157 |
| 2 | 48 |
| 2 | 68 |
| 3 | 49 |
| 3 | 56 |
| 3 | 31 |
| 8 | 170 |
| 8 | 220 |

### Beispiel 22 (Bindung an AKR1C3)

Die Bindung von Beispielverbindung 1 an das AKR1C3-Enzym wurde mittels isothermer Titrationskalorimetrie gemessen. Der Grad der Affinität einer gegebenen Verbindung kann mit der Dissoziationskonstante (K_{D}-Wert) angegeben werden. Humanes AKR1C3-Enzym wurde wie in Beispiel 20 beschrieben hergestellt. Für ITC-Experimente wurde humanes AKR1C3 Protein mittels Gel-Permeation in einen PBS-Puffer bestehend aus 137 mM NaCl, 2.7 mM KCl, 10 mM Na₂HPO₄, 2mM KH₂PO₄ und 1 mM Tris(2-carboxyethyl)phosphinhydrochlorid überführt. Die Verbindungen wurden in 100% DMSO in einer Konzentration von 10 mM gelöst und anschließend in PBS-Puffer verdünnt. Isotherme Titrationskalorimetrie-Experimente (ITC-Experimente) wurden mit einem ITC200 Titrationskalorimeter (GE Healthcare, Northampton, MA, USA) bei einer Temperatur von 25 °C durchgeführt. Die Enthalpieänderung, die aus seiner Injektion des Proteins resultiert, wurde durch Integration des kalorimetrischen Signals bestimmt. Die Daten wurden mit Origin 7.0 (GE Healthcare, Northampton, MA, USA) analysiert. Verdünnungswärmen wurden anhand der letzten Injektionen der betreffenden Titration bestimmt und vor dem Kurven-Fitten subtrahiert. Die Proteinkonzentration in der Injektionsspritze lag im Bereich von 80 bis 120 µM und die Konzentration der zu messenden Verbindung lag im Bereich von 5 bis 20 µM.

Für Beispielverbindung 1 wurde ein K_{D}-Wert von 230 nanomolar und eine exotherme Bindungsenthalpie von - 1500 kcal/mol bestimmt.

## Patentansprüche

1. Verbindungen der Formel (I) 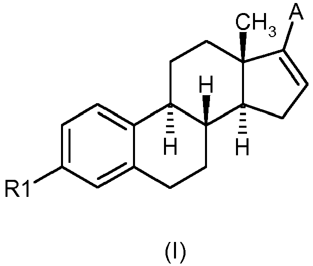 worin
A Pyridin-3-yl, Pyrimidin-5-yl, Pyrimidin-4-yl, Pyrazin-2-yl, Pyridazin-4-yl, Pyridazin-3-yl, gegebenenfalls einfach oder zweifach substituiert mit Fluor, Chlor, Nitril, Hydroxyl, Carboxyl, Trifluormethyl, Pentafluorethyl, Methoxy, Ethoxy, Trifluormethoxy, -OCH₂CF₃, -SO₂CH₃, -SO₂CH₂CH₃, -(C=O)CH₃, C₁-C₄-Alkyl, -CH₂OH, -C(CH₃)₂OH, -CONH₂, -(C=O)NHCH₃, -(C=O)NHCH₂CH₃, -(C=O)N(CH₃)₂, -SO₂NH₂, -SO₂NHCH₃, -SO₂N(CH₃)₂,
R1 -O-CR^{a}R^{b} -Y, mit
R^{a} und R^{b} unabhängig voneinander Wasserstoff, Methyl, Ethyl, Propyl, Isopropyl, Phenyl, 4-Fluorphenyl, 3-Fluorphenyl, 2-Fluorphenyl, CH₃- O-CH₂-,
-CH₂CF₃ oder
R^{a} und R^{b} gemeinsam -(CH₂)ₙ- mit n = 2, 3, 4 oder 5 oder
R^{a} und R^{b} gemeinsam -CH₂-O-CH₂-, -CH₂-NH-CH₂-, -CH₂-N(CH₃)- CH₂-,
-CH₂CH₂-O-CH₂CH₂-, -CH₂CH₂-NH-CH₂CH₂-, -CH₂CH₂-N(CH₃)- CH₂CH₂-
-O-CR^{c}R^{d}-CR^{e}R^{f}-Y, mit
R^{c}, R^{d}, R^{e} R^{f} Wasserstoff oder
R^{e}, R^{f} Wasserstoff und R^{c}, R^{d} unabhängig voneinander Methyl, Ethyl oder gemeinsam -(CH₂)ₙ- mit n = 2, 3, 4, 5 oder gemeinsam -CH₂-O- CH₂- oder
-CH₂CH₂-O-CH₂CH₂- oder
R^{c}, R^{d} Wasserstoff und R^{e}, R^{f} unabhängig voneinander Methyl, Ethyl, CF₃CH₂- oder gemeinsam -(CH₂)n- mit n = 2, 3, 4, 5, -CH₂CH₂-O-CH₂CH₂-, -
CH₂CH₂-NH-CH₂CH₂-, -CH₂CH₂-N(CH₃)-CH₂CH₂-, -CH₂-O-CH₂-, -CH₂-NH-CH₂-, -CH₂-N(CH₃)-CH₂- oder
R^{d}, R^{e}, R^{f} Wasserstoff und R^{c} Methyl, Ethyl, Trifluormethyl oder
R^{c}, R^{d}, R^{f} Wasserstoff und R^{e} Methyl, Ethyl, Trifluormethyl, Hydroxyl, Methoxy, Trifluormethoxy oder
R^{d}, R^{f} Wasserstoff und R^{c}, R^{e} unabhängig voneinander Methyl, Ethyl, Trifluormethyl,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂-CH(OH)-CH₂-Y
-OCH₂CH₂CH₂CH₂-Y,
-CH₂-Y,
-CR^{g}R^{h}-CRⁱR^{j}-Y, mit
R^{g,} R^{h,} Rⁱ, R^{j} Wasserstoff oder
R^{g}, R^{h}, Rⁱ Wasserstoff und R^{j} Methyl, Ethyl, Trifluormethyl oder
Rⁱ, R^{j} Wasserstoff und R^{g}, R^{h} Methyl oder gemeinsam -CH₂-,-CH₂CH₂-,
-CH₂CH₂CH₂- oder
R^{g} Methyl und R^{h}, Rⁱ, R^{j} Wasserstoff,
-CH₂CH₂CH₂-Y,
-CH₂CH₂C(CH₃)₂-Y oder
-CH₂CH₂CH₂CH₂-Y und
Y -CO₂H, -OH, -(C=O)NH₂, -(C=O)NHC₁₋₄Alkyl, -S(=O)CH₃
bedeutet und deren Salze, Solvate und Solvate der Salze.

2. Verbindungen der Formel (I) nach Anspruch 1, worin
A Pyridin-3-yl, Pyrimidin-5-yl, Pyrimidin-4-yl, Pyrazin-2-yl, Pyridazin-4-yl, Pyridazin-3-yl, gegebenenfalls einfach substituiert mit Fluor, Chlor, Nitril, Hydroxyl, Carboxyl, Trifluormethyl, Methoxy, Ethoxy, Trifluormethoxy, -SO₂CH₃,
-(C=O)CH₃, C₁-C₄-Alkyl,
R1 -O-CR^{a}R^{b} -Y, mit
R^{a} und R^{b} unabhängig voneinander Wasserstoff, Methyl, Ethyl, Propyl, Isopropyl, Phenyl, CH₃-O-CH₂-, CF₃CH₂-,
-O-CR^{c}R^{d}-CR^{e}R^{f}-Y, mit
R^{c}, R^{d}, R^{e} R^{f} Wasserstoff oder
R^{c}, R^{d} Wasserstoff und Re, Rf unabhängig voneinander Methyl, Ethyl, CF₃CH₂- oder
R^{d}, R^{e}, R^{f} Wasserstoff und R^{c} Methyl, Ethyl oder
R^{c}, R^{d}, R^{f} Wasserstoff und R^{e} Methyl, Ethyl,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂-CH(OH)-CH2-Y oder
-CR^{g}R^{h}-CRⁱR^{j}-Y, mit
R^{g,} R^{h,} Rⁱ, R^{j} Wasserstoff oder
R^{g}, R^{h}, Rⁱ Wasserstoff und R^{j} Methyl, Ethyl oder
R¹, R^{j} Wasserstoff und R^{g}, R^{h} Methyl oder
R^{g} Methyl und R^{h}, Rⁱ, R^{j} Wasserstoff und
Y -CO₂H, -OH, -(C=O)NH₂, -(C=O)NHC₁₋₄Alkyl
bedeutet und deren Salze, Solvate und Solvate der Salze.

3. Verbindungen der Formel (I) nach Anspruch 1, worin
A Pyridin-3-yl, Pyrimidin-5-yl, Pyridazin-4-yl, gegebenenfalls einfach substituiert mit Fluor, Carboxyl, Trifluormethyl, Methyl
R1 -O-CR^{a}R^{b} -Y, mit
R^{a} und R^{b} Wasserstoff oder R^{a} Wasserstoff und R^{b} Methyl, Ethyl ,
Phenyl oder CH₃-O-CH₂--O-CR^{c}R^{d}-CR^{e}R^{f}-Y, mit
R^{c}, R^{d} Wasserstoff und R^{e}, R^{f} Methyl
-O-CH₂CH₂CH₂-Y,
-O-CH₂C(CH₃)₂CH₂-Y,
-O-CH₂-CH(OH)-CH2-Y oder
-CH₂-CH₂-Y und
Y -CO₂H, -OH, -(C=O)NH₂, -(C=O)NHC₁₋₄Alkyl
bedeutet und deren Salze, Solvate und Solvate der Salze.

4. Verbindungen der Formel (I) nach Anspruch 1, worin
A 5-Fluorpyridin-3-yl, 5-(Trifluormethyl)pyridin-3-yl, 5-Carboxypyridin-3-yl, 6-Methylpyridin-3-yl, Pyrimidin-5-yl, 6-Methylpyridazin-4-yl und
R1 -O-CR^{a}R^{b} -CO₂H,
-O-CR^{a}R^{b}-(C=O)NH₂,
-O-CR^{a}R^{b}-(C=O)NHCH₂CH₃ mit
R^{a} und R^{b} Wasserstoff oder
R^{a} Wasserstoff und R^{b} Methyl, Ethyl, Phenyl, CH₃OCH₂-,
-O-CR^{c}R^{d}-CR^{e}R^{f}-CO₂H mit
R^{c}, R^{d} Wasserstoff und R^{e}, R^{f} Methyl,
-O-CH₂CH₂CH₂-OH,
-O-CH₂CH₂CH₂-CO₂H,
-O-CH₂C(CH₃)₂CH₂-OH oder
-O-CH₂-CH(OH)-CH₂-OH
bedeutet und deren Salze, Solvate und Solvate der Salze.

5. Verbindungen der Formel (I) nach Anspruch 1, nämlich 3-[17-(5-Fluorpyridin-3-yl)estra-1,3,5(10),16-tetraen-3-yl]propansäure ({17-[5-(Trifluormethyl)pyridin-3-yl]estra-1,3,5(10),16-tetraen-3-yl}oxy)essigsäure {[17-(5-Fluorpyridin-3-yl)estra-1,3,5(10),16-tetraen-3-yl]oxy}essigsäure {[17-(6-Methylpyridin-3-yl)estra-1,3,5(10),16-tetraen-3-yl]oxy}essigsäure {[17-(Pyrimidin-5-yl)estra-1,3,5(10),16-tetraen-3-yl]oxy}essigsäure 5-[3-(Carboxymethoxy)estra-1,3,5(10),16-tetraen-17-yl]nicotinsäure {[17-(6-Methylpyridazin-4-yl)estra-1,3,5(10),16-tetraen-3-yl]oxy}essigsäure 3-{[17-(5-Fluorpyridin-3-yl)estra-1,3,5(10),16-tetraen-3-yl]oxy}-2,2-dimethylpropansäure 4-{[17-(5-Fluorpyridin-3-yl)estra-1,3,5(10),16-tetraen-3-yl]oxy}butansäure 2-{[17-(5-Fluorpyridin-3-yl)estra-1,3,5(10),16-tetraen-3-yl]oxy}propansäure 2-{[17-(5-Fluorpyridin-3-yl)estra-1,3,5(10),16-tetraen-3-yl]oxy}butansäure 2-{[17-(5-Fluorpyridin-3-yl)estra-1,3,5(10),16-tetraen-3-yl]oxy}-3-methoxypropansäure 2-{[17-(3-Pyridyl)estra-1,3,5(10),16-tetraen-3-yl]oxy}propansäure 2-{[17-(5-Fluorpyridin-3-yl)estra-1,3,5(10),16-tetraen-3-yl]oxy}propanamid 3-{[17-(5-Fluorpyridin-3-yl)estra-1,3,5(10),16-tetraen-3-yl]oxy}propan-1-ol 3-{[17-(5-Fluorpyridin-3-yl)estra-1,3,5(10),16-tetraen-3-yl]oxy}-2,2-dimethylpropan-1-ol 3-{[17-(5-Fluorpyridin-3-yl)estra-1,3,5(10),16-tetraen-3-yl]oxy}propan-1,2-diol {[17-(5-Fluorpyridin-3-yl)estra-1,3,5(10),16-tetraen-3-yl]oxy}(phenyl)essigsäure N-Ethyl-2-({17-[5-(trifluormethyl)pyridin-3-yl]estra-1,3,5(10),16-tetraen-3-yl}oxy)acetamid
und deren Salze, Solvate und Solvate der Salze.

6. Verbindung der Formel (I), wie in einem der Ansprüche 1 bis 5 definiert, zur Behandlung und/oder Prophylaxe von Krankheiten.

7. Verbindung der Formel (I), wie in einem der Ansprüche 1 bis 5 definiert, zur Verwendung in einem Verfahren zur Behandlung und/oder Prophylaxe der Endometriose, von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes.

8. Arzneimittel enthaltend eine Verbindung der Formel (I), wie in einem der Ansprüche 1 bis 5 definiert, in Kombination mit einem oder mehreren weiteren Wirkstoff(en).

9. Arzneimittel enthaltend eine Verbindung der Formel (I), wie in einem der Ansprüche 1 bis 5 definiert, in Kombination mit einem inerten, nichttoxischen, pharmazeutisch geeigneten Hilfsstoff.

10. Arzneimittel nach Anspruch 8 oder 9 zur Behandlung und/oder Prophylaxe der Endometriose, von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes.

## Claims

1. Compounds of the formula (I) 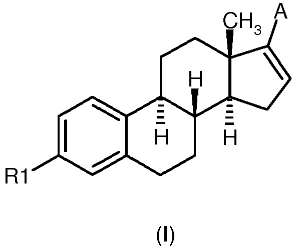 in which
A represents pyridin-3-yl, pyrimidin-5-yl, pyrimidin-4-yl, pyrazin-2-yl, pyridazin-4-yl, pyridazin-3-yl, optionally mono- or disubstituted by fluorine, chlorine, nitrile, hydroxyl, carboxyl, trifluoromethyl, pentafluoroethyl, methoxy, ethoxy, trifluoromethoxy, -OCH₂CF₃, -SO₂CH₃, -SO₂CH₂CH₃, -(C=O)CH₃, C₁-C₄-Alkyl, -CH₂OH, -C(CH₃)₂OH,-CONH₂, -(C=O)NHCH₃, -(C=O)NHCH₂CH₃, -(C=O)N(CH₃)₂, -SO₂NH₂,-SO₂NHCH₃, -SO₂N(CH₃)₂,
R1 represents -O-CR^{a}R^{b} -Y where
R^{a} and R^{b} independently of one another represent represent hydrogen, methyl, ethyl, propyl, isopropyl, phenyl, 4-fluorophenyl, 3-fluorophenyl, 2-fluorophenyl, CH₃-O-CH₂-, -CH₂CF₃ or R^{a} and R^{b} together represent -(CH₂)ₙ-where n = 2, 3, 4 or 5 or R^{a} and R^{b} together represent -CH₂-O-CH₂-, -CH₂-NH-CH₂-, -CH₂-N(CH₃)-CH₂-, -CH₂CH₂-O-CH₂CH₂-, -CH₂CH₂-NH-CH₂CH₂-, -CH₂CH₂-N(CH₃)-CH₂CH₂-
-O-CR^{c}R^{d}-CR^{e}R^{f}-Y where
R^{c}, R^{d}, R^{e} R^{f} represent hydrogen or R^{e}, R^{f} represent hydrogen and R^{c}, R^{d} independently of one another represent methyl, ethyl or together represent-(CH₂)ₙ- where n = 2, 3, 4, 5 or together represent -CH₂-O-CH₂- or -CH₂CH₂-O-CH₂CH₂-or
R^{c}, R^{d} represent hydrogen and R^{e}, R^{f} independently of one another represent methyl, ethyl, CF₃CH₂- or together represent -(CH₂)n- where n = 2, 3, 4, 5, -CH₂CH₂-O-CH₂CH₂-, -CH₂CH₂-NH-CH₂CH₂-, -CH₂CH₂-N(CH₃)-CH₂CH₂-, -CH₂-O-CH₂-, -CH₂-NH-CH₂-, -CH₂-N(CH₃)-CH₂- or R^{d}, R^{e}, R^{f} represent hydrogen and R^{c} represents methyl, ethyl, trifluoromethyl or R^{c}, R^{d}, R^{f} represent hydrogen and R^{e} represents methyl, ethyl, trifluoromethyl, hydroxyl, methoxy, trifluoromethoxy or R^{d}, R^{f} represent hydrogen and R^{c}, R^{e} independently of one another represent methyl, ethyl, trifluoromethyl,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂-CH(OH)-CH2-Y
-OCH₂CH₂CH₂CH₂-Y,
-CH₂-Y,
-CR^{g}R^{h}-CRⁱR^{j}-Y where
R^{g,} R^{h,} Rⁱ, T^{j} represent hydrogen or R^{g}, R^{h}, Rⁱ represent hydrogen and R^{j} represents methyl, ethyl, trifluoromethyl or Rⁱ, R^{j} represent hydrogen and R^{g}, R^{h} represent methyl or together represent-CH₂-, -CH₂CH₂-, -CH₂CH₂CH₂- or R⁹ represents methyl and R^{h}, Rⁱ, R^{j} represent hydrogen,
-CH₂CH₂CH₂-Y,
-CH₂CH₂C(CH₃)₂-Y or
-CH₂CH₂CH₂CH₂-Y and
Y represents -CO₂H, -OH, -(C=O)NH₂, -(C=O)NHC₁-₄-alkyl, -S(=O)CH₃
and their salts, solvates and solvates of the salts.

2. Compounds of the formula (I) according to Claim 1, in which
A represents pyridin-3-yl, pyrimidin-5-yl, pyrimidin-4-yl, pyrazin-2-yl, pyridazin-4-yl, pyridazin-3-yl, optionally monosubstituted by fluorine, chlorine, nitrile, hydroxyl, carboxyl, trifluoromethyl, methoxy, ethoxy, trifluoromethoxy,
-SO₂CH₃,
-(C=O)CH₃, C₁-C₄-alkyl,
R1 represents -O-CR^{a}R^{b} -Y where
R^{a} and R^{b} independently of one another represent represent hydrogen, methyl, ethyl, propyl, isopropyl, phenyl, CH₃-O-CH₂-, CF₃CH₂-,
-O-CR^{c}R^{d}-CR^{e}R^{f}-Y where
R^{c}, R^{d}, R^{e} R^{f} represent hydrogen or
R^{c}, R^{d} represent hydrogen and Re, Rf independently of one another represent methyl, ethyl, CF₃CH₂- or
R^{d}, R^{e}, R^{f} represent hydrogen and R^{c} represents methyl, ethyl or
R^{c}, R^{d}, R^{f} represent hydrogen and R^{e} represents methyl, ethyl,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂-CH(OH)-CH2-Y or
-CR^{g}R^{h}-CRⁱR^{j}-Y where
R^{g}, R^{h}, Rⁱ, R^{j} represent hydrogen or
R^{g}, R^{h}, Rⁱ represent hydrogen and R^{j} represents methyl, ethyl or
Rⁱ, R^{j} represent hydrogen and R^{g}, R^{h} represents methyl or
R⁹ represents methyl and R^{h}, Rⁱ, R^{j} represent hydrogen and
Y represents -CO₂H, -OH, -(C=O)NH₂, -(C=O)NHC₁-₄-alkyl
and their salts, solvates and solvates of the salts.

3. Compounds of the formula (I) according to Claim 1, in which
A represents pyridin-3-yl, pyrimidin-5-yl, pyridazin-4-yl, optionally monosubstituted by fluorine, carboxyl, trifluoromethyl, methyl
R1 represents -O-CR^{a}R^{b} -Y where
R^{a} and R^{b} represent hydrogen or R^{a} represents hydrogen and R^{b} represents methyl, ethyl, phenyl or CH₃-O-CH₂-
-O-CR^{c}R^{d}-CR^{e}R^{f}-Y where
R^{c}, R^{d} represent hydrogen and R^{e}, R^{f} represent methyl
-O-CH₂CH₂CH₂-Y,
-O-CH₂C(CH₃)₂CH₂-Y,
-O-CH₂-CH(OH)-CH2-Y or
-CH₂-CH₂-Y and
Y represents -CO₂H, -OH, -(C=O)NH₂, -(C=O)NHC₁-₄-alkyl
and their salts, solvates and solvates of the salts.

4. Compounds of the formula (I) according to Claim 1, in which
A represents 5-fluoropyridin-3-yl, 5-(trifluoromethyl)pyridin-3-yl, 5-carboxy-pyridin-3-yl, 6-methylpyridin-3-yl, pyrimidin-5-yl, 6-methylpyridazin-4-yl and
R1 represents -O-CR^{a}R^{b} -CO₂H,
-O-CR^{a}R^{b}- (C=O)NH₂,
-O-CR^{a}R^{b}-(C=O)NHCH₂CH₃ where
R^{a} and R^{b} represent hydrogen or
R^{a} represents hydrogen and R^{b} represents methyl, ethyl, phenyl, CH₃OCH₂-,
-O-CR^{c}R^{d}-CR^{e}R^{f}-CO₂H where
R^{c}, R^{d} represent hydrogen and R^{e}, R^{f} represent methyl,
-O-CH₂CH₂CH₂-OH, -O-CH₂CH₂CH₂-CO₂H, -O-CH₂C(CH₃)₂CH₂-OH or -O-CH₂-CH(OH)-CH₂-OH
and their salts, solvates and solvates of the salts.

5. Compounds of the formula (I) according to Claim 1, namely 3-[17-(5-fluoropyridin-3-yl)estra-1,3,5(10),16-tetraen-3-yl]propanoic acid ({17-[5-(trifluoromethyl)pyridin-3-yl]estra-1,3,5(10),16-tetraen-3-yl}oxy)acetic acid {[17-(5-fluoropyridin-3-yl)estra-1,3,5(10),16-tetraen-3-yl]oxy}acetic acid {[17-(6-methylpyridin-3-yl)estra-1,3,5(10),16-tetraen-3-yl]oxy}acetic acid {[17-(pyrimidin-5-yl)estra-1,3,5(10),16-tetraen-3-yl]oxy}acetic acid 5-[3-(carboxymethoxy)estra-1,3,5(10),16-tetraen-17-yl]nicotinic acid {[17-(6-methylpyridazin-4-yl)estra-1,3,5(10),16-tetraen-3-yl]oxy}acetic acid 3-{[17-(5-fluoropyridin-3-yl)estra-1,3,5(10),16-tetraen-3-yl]oxy}-2,2-dimethylpropanoic acid 4-{[17-(5-fluoropyridin-3-yl)estra-1,3,5(10),16-tetraen-3-yl]oxy}butanoic acid 2-{[17-(5-fluoropyridin-3-yl)estra-1,3,5(10),16-tetraen-3-yl]oxy}propanoic acid 2-{[17-(5-fluoropyridin-3-yl)estra-1,3,5(10),16-tetraen-3-yl]oxy}butanoic acid 2-{[17-(5-fluoropyridin-3-yl)estra-1,3,5(10),16-tetraen-3-yl]oxy}-3-methoxypropanoic acid 2-{[17-(3-pyridyl)estra-1,3,5(10),16-tetraen-3-yl]oxy}propanoic acid 2-{[17-(5-fluoropyridin-3-yl)estra-1,3,5(10),16-tetraen-3-yl]oxy}propanamide 3-{[17-(5-fluoropyridin-3-yl)estra-1,3,5(10),16-tetraen-3-yl]oxy}propan-1-ol 3-{[17-(5-fluoropyridin-3-yl)estra-1,3,5(10),16-tetraen-3-yl]oxy}-2,2-dimethylpropan-1-ol 3-{[17-(5-fluoropyridin-3-yl)estra-1,3,5(10),16-tetraen-3-yl]oxy}propan-1,2-diol {[17-(5-fluoropyridin-3-yl)estra-1,3,5(10),16-tetraen-3-yl]oxy}(phenyl)acetic acid N-ethyl-2-({17-[5-(trifluoromethyl)pyridin-3-yl]estra-1,3,5(10),16-tetraen-3-yl}oxy)acetamide and their salts, solvates and solvates of the salts.

6. Compound of the formula (I) as defined in any of Claims 1 to 5 for the treatment and/or prophylaxis of diseases.

7. Compound of the formula (I) as defined in any of Claims 1 to 5 for use in a method for the treatment and/or prophylaxis of diseases of endometriosis, of uterine leiomyomas, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkin lymphomas, of chronic obstructive pulmonary disease (COPD), of adiposity or of inflammatory pain.

8. Medicament comprising a compound of the formula (I) as defined in any of Claims 1 to 5 in combination with one or more further active compound(s).

9. Medicament comprising a compound of the formula (I) as defined in any of Claims 1 to 5 in combination with an inert non-toxic pharmaceutically suitable auxiliary.

10. Medicament according to Claim 8 or 9 for the treatment and/or prophylaxis of endometriosis, of uterine leiomyomas, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkin lymphomas, of chronic obstructive pulmonary disease (COPD), of adiposity or of inflammatory pain.

## Revendications

1. Composés de formule (I) 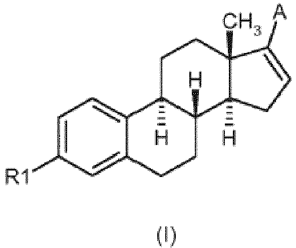 dans laquelle
A représente un groupe pyridin-3-yle, pyrimidin-5-yle, pyrimidin-4-yle, pyrazin-2-yle, pyridazin-4-yle, pyridazin-3-yle, éventuellement une ou deux fois substitué par fluoro, chloro, nitrile, hydroxy, carboxy, trifluorométhyle, pentafluoro-éthyle, méthoxy, éthoxy, trifluorométhoxy, -OCH₂CF₃, -SO₂CH₃, -SO₂CH₂CH₃, -(C=O)CH₃, alkyle en C₁-C₄, -CH₂OH, -C(CH₃)₂OH, -CONH₂, -(C=O)NHCH₃, -(C=O)NHCH₂CH₂, -(C=O)N(CH₃)₂, -SO₂NH₂, -SO₂NHCH₃, -SO₂N(CH₃)₂,
R1 représente un groupe -O-CR^{a}R^{b}-Y, où
R^{a} et R^{b} représentent indépendamment l'un de l'autre un atome d'hydrogène, le groupe méthyle, éthyle, propyle, isopropyle, phényle, 4-fluorophényle, 3-fluorophényle, 2-fluorophényle, CH₃-O-CH₂-, -CH₂CF₃ ou
R^{a} et R^{b} ensemble représentent un groupe -(CH₂)ₙ-, où n = 2, 3, 4 ou 5 ou
R^{a} et R^{b} ensemble représentent le groupe -CH₂-O-CH₂-, -CH₂-NH-CH₂-, -CH₂-N(CH₃)-CH₂-, -CH₂CH₂-O-CH₂CH₂-, -CH₂CH₂-NH-CH₂CH₃-, -CH₂CH₂-N(CH₃)-CH₂CH₂-
un groupe -O-CR^{c}R^{d}-CR^{e}R^{f}-Y, où
R^{c}, R^{d}, R^{e}, R^{f} représentent un atome d'hydrogène ou
R^{e}, R^{f} représentent un atome d'hydrogène et R^{c}, R^{d} représentent indépendamment l'un de l'autre le groupe méthyle, éthyle, ou ensemble un groupe -(CH₂)ₙ-, où n = 2, 3, 4, 5 ou ensemble un groupe -CH₂-O-CH₂- ou -CH₂CH₂-O-CH₂CH₂- ou
R^{c}, R^{d} représentent un atome d'hydrogène et R^{e}, R^{f} représentent indépendamment l'un de l'autre le groupe méthyle, éthyle, CF₃CH₂- ou ensemble un groupe -(CH₂)ₙ-, où n = 2, 3, 4, 5, -CH₂CH₁-O-CH₂CH₂-, -
CH₂CH₂-NH-CH₂CH₂-, -CH₂CH₂-N(CH₃)-CH₂CH₂-, -CH₂-O-CH₂-, -CH₂-NH-CH₂-, -CH₂-N(CH₃)-CH₂- ou
R^{d}, R^{e}, R^{f} représentent un atome d'hydrogène et R^{c} représente le groupe méthyle, éthyle, trifluorométhyle ou
R^{c}, R^{d}, R^{f} représentent un atome d'hydrogène et R^{e} représente le groupe méthyle, éthyle, trifluorométhyle, hydroxy, méthoxy, trifluorométhoxy ou
R^{d}, R^{f} représentent un atome d'hydrogène et
R^{c}, R^{e} représentent indépendamment l'un de l'autre le groupe méthyle, éthyle, trifluorométhyle,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂-CH(OH)-CH₂-Y
-OCH₂CH₂CH₂CH₂-Y,
-CH₂-Y,
-CR^{g}R^{h}-CRⁱR^{j}-Y, où
R^{g}, R^{h}, Rⁱ, R^{j} représentent un atome d'hydrogène ou
R^{g}, R^{h}, Rⁱ représentent un atome d'hydrogène et R^{j} représente le groupe méthyle, éthyle, trifluorométhyle ou
Rⁱ, R^{j} représentent un atome d'hydrogène et R^{g}, R^{h} représentent le groupe méthyle ou ensemble -CH₂-, -CH₂CH₂-, -CH₂CH₂CH₂- ou
R^{g} représente le groupe méthyle et R^{h}, Rⁱ, R^{j} représentent un atome d'hydrogène,
-CH₂CH₂CH₂-Y,
-CH₂CH₂C(CH₃)₂-Y ou
-CH₂CH₂CH₂CH₂-Y et
Y représente -CO₂H, -OH, -(C=O)NH₂, -(C=O)NH-alkyle(C₁-C₄), -S(=O)C₃;
et leurs sels, produits de solvatation et produits de solvatation des sels.

2. Composés de formule (I) selon la revendication 1, dans lesquels
A représente un groupe pyridin-3-yle, pyrimidin-5-yle, pyrimidin-4-yle, pyrazin-2-yle, pyridazin-4-yle, pyridazin-3-yle, éventuellement une fois substitué par fluoro, chloro, nitrile, hydroxy, carboxy, trifluorométhyle, méthoxy, éthoxy, trifluorométhoxy, -SO₂CH₃,
-(C=O)CH₃, alkyle en C₁-C₄,
R1 représente un groupe -O-CR^{a}R^{b}-Y, où
R^{a} et R^{b} représentent indépendamment l'un de l'autre un atome d'hydrogène, le groupe méthyle, éthyle, propyle, isopropyle, phényle, CH₃-O-CH₂-, CF₃CH₂-,
un groupe -O-CR^{c}R^{d}-CR^{e}R^{f}-Y, où
R^{c}, R^{d}, R^{e}, R^{f} représentent un atome d'hydrogène ou
R^{c}, R^{d} représentent un atome d'hydrogène et
R^{e}, R^{f} représentent indépendamment l'un de l'autre le groupe méthyle, éthyle, CF₃CH₂- ou
R^{d}, R^{e}, R^{f} représentent un atome d'hydrogène
et R^{c} représente le groupe méthyle, éthyle ou
R^{c}, R^{d}, R^{f} représentent un atome d'hydrogène
et R^{e} représente le groupe méthyle, éthyle,
-O-CH₂CH₂CH₂-Y,
-O-CH₂C(CH₃)₂CH₂-Y,
-O-CH₂CH₂C(CH₃)₂-Y,
-O-CH₂CH₂CH(CH₃)-Y,
-O-CH₂CH(OH)-CH₂-Y ou -CR^{g}R^{h}-CRⁱR^{j}-Y, où
R^{g}, R^{h}, Rⁱ, R^{j} représentent un atome d'hydrogène ou
R^{g}, R^{h}, Rⁱ représentent un atome d'hydrogène et R^{j} représente le groupe méthyle, éthyle ou
Rⁱ, R^{j} représentent un atome d'hydrogène et R^{g}, R^{h} représentent le groupe méthyle ou
R^{g} représente le groupe méthyle et R^{h}, Rⁱ, R^{j} représentent un atome d'hydrogène et
Y représente -CO₂H, -OH, -(C=O)NH₂, -(C=O)NH-alkyle(C₁-C₄)
et leurs sels, produits de solvatation et produits de solvatation des sels.

3. Composés de formule (I) selon la revendication 1, dans lesquels
A représente un groupe pyridin-3-yle, pyrimidin-5-yle, pyridazin-4-yle, éventuellement une fois substitué par fluoro, carboxy, trifluorométhyle, méthyle,
R1 représente un groupe -O-CR^{a}R^{b}-Y, où
R^{a} et R^{b} représentent un atome d'hydrogène ou R^{a} représente un atome d'hydrogène et R^{b} représente le groupe méthyle, éthyle, phényle ou CH₃-O-CH₂-,
un groupe -O-CR^{c}R^{d}-CR^{e}R^{f}-Y, où
R^{c}, R^{d} représentent un atome d'hydrogène et
R^{e}, R^{f} représentent le groupe méthyle,
-O-CH₂CH₂CH₂-Y,
-O-CH₂C(CH₃)₂CH₂-Y,
-O-CH₂-CH(OH)-CH₂-Y ou
-CH₂-CH₂-Y et
Y représente -CO₂H, -OH, -(C=O)NH₂, -(C=O)NH-alkyle(C₁-C₄)
et leurs sels, produits de solvatation et produits de solvatation des sels.

4. Composés de formule (I) selon la revendication 1, dans lesquels
A représente le groupe 5-fluoropyridin-3-yle, 5-(trifluorométhyl)pyridin-3-yle, 5-carboxy-pyridin-3-yle, 6-méthylpyridin-3-yle, pyrimidin-5-yle, 6-méthylpyridazin-4-yle et
R1 représente un groupe
-O-CR^{a}R^{b}-CO₂H,
-O-CR^{a}R^{b}-(C=O)NH₂,
-O-CR^{a}R^{b}-(C=O)NHCH₂CH₃, où
R^{a} et R^{b} représentent un atome d'hydrogène ou R^{a} représente un atome d'hydrogène et R^{b} représente le groupe méthyle, éthyle, phényle, CH₃OCH₂-,
-O-CR^{c}R^{d}-CR^{e}R^{f}-CO₂H où
R^{c}, R^{d} représentent un atome d'hydrogène et
R^{e}, R^{f} représentent le groupe méthyle,
-O-CH₂CH₂CH₂-OH,
-O-CH₂CH₂CH₂-CO₂H,
-O-CH₂C(CH₃)₂CH₂-OH ou
-O-CH₂-CH(OH)-CH₂-OH
et leurs sels, produits de solvatation et produits de solvatation des sels.

5. Composés de formule (I) selon la revendication 1, à savoir
acide 3-[17-(5-fluoropyridin-3-yl)estra-1,3,5(10),16-tétraén-3-yl]propionique
acide ({17-[5-(trifluorométhyl)pyridin-3-yl]estra-1,3,5 (10),16-tétraén-3-yl}oxy)acétique
acide {[17-(5-fluoropyridin-3-yl)estra-1,3,5(10),16-tétraén-3-yl]oxy}acétique
acide {[17-(6-méthylpyridin-3-yl)estra-1,3,5(10),16-tétraén-3-yl]oxy}acétique
acide {[17-(pyrimidin-5-yl)estra-1,3,5(10),16-tétraén-3-yl]oxy}acétique
acide 5-[3-(carboxyméthoxy)estra-1,3,5(10),16-tétraén-17-yl]nicotinique
acide {[17-(6-méthylpyridazin-4-yl)estra-1,3,5(10),16-tétraén-3-yl]oxy}acétique
acide 3-{[17-(5-fluoropyridin-3-yl)estra-1,3,5(10),16-tétraén-3-yl]oxy}-2,2-diméthylpropionique acide 4-{[17-(5-fluoropyridin-3-yl)estra-1,3,5(10),16-tétraén-3-yl]oxy}butanoïque
acide 2-{[17-(5-fluoropyridin-3-yl)estra-1,3,5(10),16-tétraén-3-yl]oxy}propionique
acide 2-{[17-(5-fluoropyridin-3-yl)estra-1,3,5(10),16-tétraén-3-yl]oxy}butanoïque
acide 2-{[17-(5-fluoropyridin-3-yl)estra-1,3,5(10),16-tétraén-3-yl]oxy}-3-méthoxypropionique
acide 2-{[17-(3-pyridyl)estra-1,3,5(10),16-tétraén-3-yl]oxy}propionique
2-{[17-(5-fluoropyridin-3-yl)estra-1,3,5(10),16-tétraén-3-yl]oxy}propanamide
3-{[17-(5-fluoropyridin-3-yl)estra-1,3,5(10),16-tétraén-3-yl]oxy}propan-1-ol
3-{[17-(5-fluorpyridin-3-yl)estra-1,3,5(10),16-tétraén-3-yl]oxy}-2,2-diméthylpropan-1-ol
3-{[17-(5-fluoropyridin-3-yl)estra-1,3,5(10),16-tétraén-3-yl]oxy}propane-1,2-diol
acide {[17-(5-fluoropyridin-3-yl)estra-1,3,5(10),16-tétraén-3-yl]oxy}(phenyl)acétique
N-éthyl-2-({17-[5-(trifluorméthyl)pyridin-3-yl]estra-1,3,5(10),16-tétraén-3-yl}oxy)acétamide
et leurs sels, produits de solvatation et produits de solvatation des sels.

6. Composé de formule (I), tel que défini dans l'une quelconque des revendications 1 à 5, destiné au traitement et/ou à la prophylaxie de maladies.

7. Composé de formule (I), tel que défini dans l'une quelconque des revendications 1 à 5, destiné à l'utilisation dans un procédé pour le traitement et/ou la prophylaxie de l'endométriose, de léiomyomes utérins, de troubles hémorragiques utérins, de la dysménorrhée, du carcinome de la prostate, de l'hyperplasie de la prostate, de l'acné, de la séborrhée, de la chute des cheveux, de la maturité sexuelle précoce, du syndrome des ovaires polykystiques, du cancer du sein, du cancer du poumon, du carcinome de l'endomètre, du carcinome des cellules rénales, du carcinome de la vessie, des lymphomes non hodgkiniens, des bronchopneumopathies chroniques obstructives (BPCO), de l'obésité ou de la douleur inflammatoire.

8. Médicament contenant un composé de formule (I), tel que défini dans l'une quelconque des revendications 1 à 5, en association avec une ou plusieurs autre (s) substance(s) active(s).

9. Médicament contenant un composé de formule (I), tel que défini dans l'une quelconque des revendications 1 à 5, en association avec un adjuvant inerte, non toxique, pharmaceutiquement acceptable.

10. Médicament selon la revendication 8 ou 9, destiné au traitement et/ou à la prophylaxie de l'endométriose, de léiomyomes utérins, de troubles hémorragiques utérins, de la dysménorrhée, du carcinome de la prostate, de l'hyperplasie de la prostate, de l'acné, de la séborrhée, de la chute des cheveux, de la maturité sexuelle précoce, du syndrome des ovaires polykystiques, du cancer du sein, du cancer du poumon, du carcinome de l'endomètre, du carcinome des cellules rénales, du carcinome de la vessie, des lymphomes non hodgkiniens, des bronchopneumopathies chroniques obstructives (BPCO), de l'obésité ou de la douleur inflammatoire.
